(19)

Europäisches
Patentamt
European
Patent Office
Office européen
des brevets

(11)  **EP 4 306 585 A1**

(12)  **EUROPEAN PATENT APPLICATION**

(43) Date of publication:
 **17.01.2024 Bulletin 2024/03**

(21) Application number: **22184117.4**

(22) Date of filing: **11.07.2022**

(51) International Patent Classification (IPC):
 ***C08J 11/08*** *(2006.01)*   ***C08J 11/12*** *(2006.01)*
 ***C08J 11/14*** *(2006.01)*

(52) Cooperative Patent Classification (CPC):
 **C08J 11/08; C08J 11/12; C08J 11/14;**
 C07D 201/12; C08J 2375/08; C08J 2377/02;
 C08J 2475/08; C08J 2477/02

(84) Designated Contracting States:
 **AL AT BE BG CH CY CZ DE DK EE ES FI FR GB**
 **GR HR HU IE IS IT LI LT LU LV MC MK MT NL NO**
 **PL PT RO RS SE SI SK SM TR**
 Designated Extension States:
 **BA ME**
 Designated Validation States:
 **KH MA MD TN**

(71) Applicant: **CAP III B.V.**
 **6129 EL Urmond (NL)**

(72) Inventors:
 • **Verduyckt, Jasper**
  **6129 EL Urmond (NL)**
 • **Groothaert, Marijke Hilde Leen**
  **6129 EL Urmond (NL)**

 • **Markusse, Abraham Peter**
  **6129 EL Urmond (NL)**
 • **Roos, Peter**
  **6129 EL Urmond (NL)**
 • **Cuiper, Anna Dite**
  **6129 EL Urmond (NL)**
 • **Fu, Wenjing**
  **6129 EL Urmond (NL)**
 • **Tinge, Johan Thomas**
  **6129 EL Urmond (NL)**

(74) Representative: **Cohausz & Florack**
 **Patent- & Rechtsanwälte**
 **Partnerschaftsgesellschaft mbB**
 **Bleichstraße 14**
 **40211 Düsseldorf (DE)**

(54) **PROCESS FOR THE RECOVERY OF EPSILON-CAPROLACTAM AND POLYETHER POLYURETHANE FROM POLYAMIDE 6 AND POLYETHER POLYURETHANE COMPRISING MATERIALS**

(57)  The present invention provides a process for recovering ε-caproiactam and polyether polyurethane from polyamide 6 and polyether polyurethane comprising material in a plant, wherein the plant comprises a separation section [B], a depolymerization section [C], and a recovery section [D]. The invention further provides apparatus suitable for carrying out the above process and polyether polyurethane and ε-caprolactam obtained by the process of the invention.

FIG. 1.

EP 4 306 585 A1

**Description**

**TECHNICAL FIELD OF THE INVENTION**

The present invention relates to a process for the recovery of ε-caprolactam from polyamide 6 and polyether polyurethane comprising materials. More particularly, the present invention relates to a process for the recovery of both purified ε-caprolactam and polyether polyurethane from polyamide 6 and polyether polyurethane comprising materials on an industrial scale.

**BACKGROUND OF THE INVENTION**

ε-Caprolactam is an important organic chemical raw material, mainly used in the production of polyamide 6 (PA6), that is also known as Nylon 6 (N6) or polycaprolactam. It is well-known that ε-caprolactam can be prepared by liquid phase Beckmann rearrangement of cyclohexanone oxime in the presence of oleum, a mixture of sulfuric acid and $SO_3$ or by gas phase Beckmann rearrangement of cyclohexanone oxime in the presence of a solid catalyst. Generally, this type of ε-caprolactam is referred to as "virgin ε-caprolactam". The required cyclohexanone oxime for the formation of (virgin) ε-caprolactam can be prepared from cyclohexanone that is mainly produced from benzene (see e.g., Ullmann's Encyclopedia of Industrial Chemistry (2018) Chapter 'Caprolactam'; https://doi.org/10.1002/14356007.a05_031.pub3). Most of this benzene originates from non-renewable fossil resources like oil and coal.

Initially, pure PA6 was used for the production of textiles, including stockings. Later, in order to improve the properties of the fabrics, a wide array of blended fabrics has been developed and produced. Especially, polyether polyurethane has been incorporated into a wide range of garments. A benefit of polyether polyurethane is its significant strength and elasticity and its ability to return to the original shape after stretching and faster drying than ordinary fabrics. Improved elasticity of fabric is often obtained by applying blended yarns that are made by blending PA6 fibers and polyurethane fibers. Examples of clothes containing fabric blends of PA6 and polyether polyurethane include stretchable stockings, underwear, and sportswear. Moisture permeability and waterproofness of fabrics can be obtained by processing (e.g., coating) the surface of PA6 fibers with a polyurethane resin. These surface treated yarns are often used for the manufacturing of, e.g., raincoats, cold weather clothes, and skiwear. Polyether polyurethanes that are used for the production of garments are also often called Spandex, Lycra or elastane.

Recycling of PA6 enables the conservation of fossil resources and can add value to the circular economy. Mechanical recycling of wasted PA6, processes in which wasted PA6 is transferred into secondary raw material or into products for which (preferably) the chemical structure of the material is only minimally changed. Conversion of PA6 and depolymerization of PA6 are two forms of chemical recycling of PA6. In the conversion recycling process PA6 is broken down into oil- or gas-like feedstock that can replace newly extracted fossil feedstock. The resulting products can be used to produce chemicals including monomer ε-caprolactam. In the depolymerization recycling process PA6 is broken down into its monomer building block ε-caprolactam. Depolymerization recycling processes for PA6 are well developed for clean and rather pure wasted PA6 products. Mechanical recycling always results in downcycling, so the properties of the products made of mechanically recycled PA6 are always worse than those made of virgin PA6. Chemical recycling of wasted PA6 containing materials, however, also might allow the production of high purity ε-caprolactam with properties similar to those of virgin made ε-caprolactam that afterwards can be converted into high grade PA6. Unfortunately, chemical recycling of wasted PA6 containing materials is often hampered by the presence of impurities in wasted PA6 materials. Generally, as a consequence of the presence of these impurities, the quality of the produced ε-caprolactam is inferior compared to virgin ε-caprolactam.

Depolymerization of blends of PA6 and polyether polyurethanes has many disadvantages compared to depolymerization of rather pure PA6. These disadvantages include amongst others blockage of piping and other pieces of equipment by polyether polyurethanes and their decomposition products, inferior quality of the produced ε-caprolactam due to the presence of decomposition products of polyether polyurethanes, (strongly) reduced ε-caprolactam recovery yields, poisoning of the depolymerization catalyst and thus higher consumption of depolymerization catalyst. Another disadvantage of the addition of blends of PA6 and polyether polyurethanes to a depolymerization reactor is that the polyether polyurethanes are destroyed. Thus, the recycling of the polyether polyurethanes, which are more valuable than PA6, is not possible.

Virgin elastane fibers are produced by dry or wet spinning processes that start from an elastane spinning solution that is made up of polyurethanes in suitable solvents such as dimethylacetamide or dimethylformamide.

Direct recycling of normal (pure) elastane wasted fibers, e.g., from yarn production, by dissolution in the employed spinning solvents proofed to be unsuccessful due to the high viscosities that prevented further processing.

US6830715B1 describes a method for producing elastane threads from spinning solutions using recycled elastane material that overcomes the before mentioned viscosity issue by addition of a secondary aliphatic amine to a mixture of (cut) elastane fibers and spinning solvent. The dissolving of the (cut) elastane fibers is realized at a temperature of 60 °C to 150 °C.

In the past already several attempts have been described for the recycling of blends of PA6 and polyether polyurethanes.

JP2011088943A describes a pre-treatment method for separating polyether polyurethane from a PA6 product containing polyether polyurethane that is followed by depolymerization of the residual PA6. The pre-treatment includes heating the PA6 product containing polyether polyurethane together with a cyclic amide containing solvent at temperatures from 80 °C to the boiling point of the solvent. JP2011088943A states that the amount of the cyclic amide compound in the cyclic amide compound solvent is preferably 50% by weight or more, more preferably 85% by mass or more. JP2011088943A states further that the cyclic amide compound solvent may contain components other than the cyclic amide compound such as water and an organic solvent, and water is particularly preferable from the viewpoint of operability. The experiments described in the patent were performed with aqueous solutions that contain 50 and 85 % by weight N-methylpyrrolidone, 85 % by weight 2-pyrrolidone or 85 % by weight 2-piperidin as solvent at a temperature of 110 °C during a period of 2 hours. As a result of this treatment, the polyether polyurethane is partly decomposed and dissolved in the solvent. Consequently, the resulting PA6 product from which polyether polyurethane has been removed and from which the solution is separated by filtration, is depolymerized. However, JP2011088943A is silent about recovery of the polyether polyurethane that is partly decomposed. Neither the fate of the used solvent is described.

WO2013032408A1 describes a method of recycling polyamide fibers including polyamide 6 and polyamide 6,6 comprising polyamide and spandex. Spandex fibers are removed from the elastomeric fabric by a process comprised of: controlled thermal degradation of spandex, controlled washing treatment for removal of spandex or its degradation products from high purity polyamide by using a suitable and sustainable solvent, preferably ethanol, and the final step to remove excess solvent from the polyamide fibers. The temperature range used during thermal treatment of polyamide fibers (including polyamide 6 fiber) is 150 °C - 220 °C, preferably from 190 °C - 216 °C and preferably during a period of 0.5 to 4 hours. Thereafter, the heat-treated fabric is washed with preferably ethanol to remove the spandex and its degraded components at temperatures ranging from 5 °C to 78 °C. WO2013032408A1 is silent about the recovery of spandex and its degraded components from the washing solvent.

In view of the above, up to now no process exists for the recovery of both purified ε-caprolactam and polyether polyurethane from polyamide 6 and polyether polyurethane comprising materials on an industrial scale. The reason why no recycling processes for the recovery of both purified ε-caprolactam and polyether polyurethane from polyamide 6 and polyether polyurethane comprising materials on an industrial scale are realized lies in the fact that in all processes to bring the elastane into solution it is necessary to apply high solution temperatures, usually well above 100 °C. At these temperatures, however, degradation of elastane starts and only inferior quality material is obtained after recovery. As a consequence, the fate of the recovered elastane is often limited to incineration and land filling. Another disadvantage of dissolution processes at high temperatures is the formation of degradation products of the applied solvent, which might hinder the recovery of polyamide 6 and polyether polyurethane and the recycling of the applied solvent.

From the viewpoint of reducing carbon dioxide emission, it is of eminent importance to recycle polyamide 6 and polyether polyurethane comprising materials and thereby recovering purified ε-caprolactam and polyether polyurethane.

The raw material price of virgin polyether polyurethane is high, in general even more than two times higher than that of virgin PA6. Thus, there is a large economic incentive for the recovery and re-use (recycling) of both wasted polyether polyurethane and PA6.

A problem with the prior art processes is that they consume a large amount of energy for the recovery of dissolved elastane from solutions because the dissolved elastane is typically obtained by removing the solvent by evaporation.

Another disadvantage of the prior art is that a suitable recovery and re-use (recycling) strategy of solvent(s) used for recovery and re-use (recycling) of polyether polyurethane and PA6 from polyamide 6 and polyether polyurethane comprising materials is not provided or known.

Finally, there is a need for processes that allow the recovery of pure ε-caprolactam and polyether polyurethane from polyamide 6 and polyether polyurethane comprising materials on an industrial scale in order to process the huge amounts of polyamide 6 and polyether polyurethane comprising materials that are wasted annually.

## SUMMARY OF THE INVENTION

It is an object of the present invention, to satisfy one or more of the above-described needs and to overcome the disadvantages associated with the prior art methods.

In particular, it is an object of the present invention to provide a process for recovering ε-caprolactam from polyamide 6 and polyether polyurethane comprising materials. It is also an object of the present invention to provide a process for recovering both purified ε-caprolactam and polyether polyurethane from polyamide 6 and polyether polyurethane comprising materials.

It is a further object of the invention to provide a process for recovery of both purified ε-caprolactam and polyether polyurethane from polyamide 6 and polyether polyurethane comprising materials on an industrial scale.

It is also an object of the invention to provide a process for recovery of both purified ε-caprolactam and polyether polyurethane from polyamide 6 and polyether polyurethane comprising materials in an economically responsible manner. In this regard, it is in particular an object of the present invention to provide a process that is suitable to recover both purified ε-caprolactam and polyether polyurethane from polyamide 6 and polyether polyurethane comprising materials and that does not exceed the production costs of virgin high purity ε-caprolactam and polyether polyurethane.

It is a further object of the invention to provide a process for recovery of both purified ε-caprolactam and polyether polyurethane from polyamide 6 and polyether polyurethane comprising materials which is characterized by a significantly lower carbon footprint than a process for producing virgin polyether polyurethane, e.g., by reaction of polyether polyols and diisocyanate monomer, and ε-caprolactam via new synthesis, e.g., by Beckmann rearrangement of cyclohexanone oxime.

The invention therefore also aims to provide a process that reduces the environmental burden of wasted polyamide 6 and polyether polyurethane comprising materials.

One or more further objects may become apparent from the remainder of the description.

All or at least some of the aforementioned objects are solved or at least mitigated to a large extent by the process of claim 1, the plant of claim 13 and the products of claims 14 and 15.

Accordingly, the present invention provides a process for recovering ε-caprolactam and polyether polyurethane from a polyamide 6 and polyether polyurethane comprising material in a plant, wherein the plant comprises

- a separation section [B],
- a depolymerization section [C], and
- a recovery section [D],

and wherein the process comprises the steps of:

a) charging the polyamide 6 and polyether polyurethane comprising material to the separation section [B];
b) separating the polyamide 6 and polyether polyurethane comprising material in a polyamide 6 rich stream and a polyether polyurethane rich stream in the separation section [B] by selective dissolution of the polyether polyurethane in an organic solvent at a temperature below 100 °C, preferably at a temperature ranging from 0 °C to 100 °C, more preferably at a temperature ranging from 10 °C to 90 °C, even more preferably at a temperature ranging from 10 °C to 80 °C, and most preferably at a temperature ranging from 20 °C to 75 °C, wherein the polyether polyurethane rich stream is a solution comprising the organic solvent and polyether polyurethane;
c.1) discharging the polyamide 6 rich stream from separation section [B] and charging the polyamide 6 rich stream to depolymerization section [C] wherein the polyamide 6 content of the polyamide 6 rich stream is at least 85 % by weight on dry-weight basis;
c.2) depolymerizing the polyamide 6 in the polyamide 6 rich stream in the depolymerization section [C] at a temperature ranging from 180 °C to 400 °C so that an ε-caprolactam comprising stream is obtained and discharging the obtained ε-caprolactam comprising stream from the depolymerization section [C];
c.3) recovering crude ε-caprolactam from said ε-caprolactam comprising stream in the recovery section [D];
d.1) recovering polyether polyurethane from the polyether polyurethane rich stream in the separation section [B]; and
d.2) discharging said recovered polyether polyurethane from the separation section [B] wherein the polyether polyurethane content of the discharged stream is at least 85 % by weight on dry-weight basis.

It was surprising that combining the special sequence of processing steps and process conditions according to the invention, i.e., the sequence of the above-defined separation, depolymerization, and recovery steps, allows to recover ε-caprolactam from polyamide 6 and polyether polyurethane comprising material in high yields and in a straightforward and economically reasonable manner. The process of the invention is economically reasonable and advantageous from several points of view. Firstly, the process of the invention is suitable for a large variety of polyamide 6 and polyether polyurethane comprising materials that can, e.g., differ in their overall composition such as their polyamide 6 content. Secondly, the process of the invention allows to effectively separate polyamide 6 from polyether polyurethane compounds so that high-grade polyamide 6 can be obtained. Thirdly, the process of the invention is so effective that the obtained polyamide 6 can be depolymerized to high-grade ε-caprolactam in high yields. Fourthly, the process of the invention allows the recovery of polyether polyurethane that can be re-used to replace polyether polyurethane produced by *de novo* synthesis, e.g., by reaction of polyether polyols and diisocyanate monomer. Fifthly, the process of the invention allows the recovery of ε-caprolactam from polyamide 6 and polyether polyurethane comprising materials on an industrial scale in order to process the huge amounts of polyamide 6 and polyether polyurethane comprising materials that are currently wasted. Lastly, the process of the invention allows to produce ε-caprolactam with a significantly lower carbon footprint compared to ε-caprolactam produced by *de novo* synthesis of ε-caprolactam, e.g., by the Beckmann rearrangement of cyclohexanone oxime. The process of the invention allows to process polyamide 6 and polyether polyurethane comprising materials efficiently and to reduce the environmental burden of said materials. In particular, the process of the invention allows the production of polyether polyurethane with a carbon footprint of less than 1 kg $CO_2$ per kg polyether polyurethane, which is a substantial improvement compared to the 4.8 kg $CO_2$ per kg polyether polyurethane associated with the production of "virgin" polyether polyurethane obtained by chemical synthesis. In particular, the process of the invention allows the production of purified ε-caprolactam with a carbon footprint of less than 3 kg $CO_2$ per kg purified ε-caprolactam, which is a substantial improvement compared to the 6.4 to 7.5 kg $CO_2$ per kg ε-caprolactam associated with the production of "virgin" ε-caprolactam obtained from a Beckmann rearrangement of cyclohexanone oxime.

Next to the process of the invention, the present invention also provides a plant, in particular an industrial-scale plant, for the production of purified ε-caprolactam and polyether polyurethane from polyamide 6 and polyether polyurethane comprising material, wherein the chemical plant comprises

- optionally, a pre-treatment section [A],
- a separation section [B],
- a depolymerization section [C],
- a recovery section [D], and
- optionally, a purification section [E], and

wherein the chemical plant is configured for carrying out the process of the invention.

The present invention also provides ε-caprolactam obtained via depolymerization of polyamide 6 that is produced from polyamide 6 and polyether polyurethane comprising material according to a process of the invention, wherein the ε-caprolactam has a product carbon footprint of less than 3.0 kg $CO_2$ per kg purified ε-caprolactam.

The present invention also provides polyether polyurethane obtained via separation from polyamide 6 and polyether polyurethane comprising material according to a process of the invention, wherein the polyether polyurethane has a product carbon footprint of less than 1.0 kg $CO_2$ per kg polyether polyurethane.

Advantageous embodiments of the invention are indicated in the dependent claims and are explained in more detail in the following.

## DETAILED DESCRIPTION OF THE INVENTION

The polyamide 6 and polyether polyurethane comprising material

The process of the invention uses a polyamide 6 and polyether polyurethane comprising material as starting material. Typically, polyamide 6 and polyether polyurethane comprising materials are solid materials, in particular fibers, yarns (i.e., strands of fibers that are spun together) or fiber-based fabrics, whereby the fibers are a blend of PA6 fibers and polyurethane fibers, or whereby the fibers are obtained by coating the surface of PA6 fibers with a polyether polyurethane resin.

The polyamide 6 and polyether polyurethane comprising material can be both of pre-consumer and post-consumer origin. The nylon 6 and polyether polyurethane comprising material can be a mixture of different nylon 6 and polyether polyurethane comprising materials or a mixture of one or more nylon 6 and polyether polyurethane comprising materials with one or more different materials.

Polyamide 6 and polyether polyurethane comprising materials can contain additional components. Such components can be added, e.g., during the polymerization, the formation of the fibers or afterwards to achieve a desired property variation. These compounds include, e.g., brightening agents, stiffening agents, anti-static lubricants, colorants, brightening agents, spin finish, agents for surface smoothness, anti-oxidation agents, UV stabilizers, and so on. The quantities of these additional components are dependent on the application of the polyamide 6 and polyether polyurethane comprising material.

The weight to weight ratio of polyamide 6 to polyether polyurethane in polyamide 6 and polyether polyurethane comprising material can vary. Preferably, the weight to weight ratio of polyamide 6 to polyether polyurethane ranges from ca. 1 : 1 to ca. 100 : 1, most preferably from ca. 3 : 1 to ca. 20 : 1.

Polyurethanes are produced by reacting an isocyanate containing two or more isocyanate groups per molecule with a polyol containing on average two or more hydroxyl groups per molecule in the presence of a catalyst or by activation with ultraviolet light. The main ingredients to make a polyurethane are di- and tri-isocyanates and polyols. Diisocyanate monomer methylene diphenyl diisocyanate (MDI) is the most commonly used isocyanate for the production of polyurethanes for garment applications.

Polyether polyurethanes are made by reaction of isocyanates with polyether polyols. Polyether polyols are generally made by polymerizing a cyclic ether compound onto an initiator compound. The cyclic ethers most commonly used in polyether polyol production are ethylene oxide, propylene oxide and 1,4-butylene oxide or tetrahydrofuran (to produce poly(butylene oxide) polyols). Poly(butylene oxide) polyols are used mostly in applications in which highly hydrophobic characteristics are needed.

The use of the term "ca." or "about" in connection with numerical values herein indicates that the numerical values may be affected by measurement errors which typically change the numerical values by not more than ± 5%. Numerical values disclosed herein together with the term "ca." or "about" are also meant to be disclosed as such, i.e., without the term "about". Further, numerical ranges as stated herein are meant to include and disclose each and every value within that range. All upper and lower end points of ranges for the same parameter disclosed herein are combinable with each other. All ranges for different parameters disclosed herein are combinable with each other. In particular, the ranges of different or the same "preference level" are particularly compatible with each other. As used in the present disclosure and claims, the singular forms "a," "an," and "the" include plural forms, especially in the sense of "one or more", unless the context clearly dictates otherwise.

Possible pre-treatment steps

Before being subjected to step a) of the process of the invention, the polyamide 6 and polyether polyurethane comprising material can undergo a pre-treatment in pre-treatment section [A], in particular size reduction in a mechanical size reduction section and/or a cleaning in a cleaning section. Preferably, the process of the invention is performed in a plant that further comprises a pre-treatment section [A], and wherein prior to step a) the polyamide 6 and polyether polyurethane comprising material is subjected to a pre-treatment in a pre-treatment section [A], in particular to a cleaning in a cleaning section and/or to a mechanical size reduction in a mechanical size reduction section. This has the advantage that the polyamide 6 and polyether polyurethane comprising products that are charged to the separation section [B] are less contaminated with foreign materials, which improves the yield and the purity of the ε-caprolactam and the polyether polyurethane that are produced in the plant of the invention configured to carry out the process of the invention. Another advantage is that polyamide 6 and polyether polyurethane comprising products that are size reduced can be more easily handled.

The dimensions and shapes of (wasted) polyamide 6 and polyether polyurethane comprising products are very much depending on the exact application. (Wasted) polyamide 6 and polyether polyurethane comprising products range from separate fibers and yarns (optionally presented in bobbins) to (wasted) woven fabrics and (used) garments. (Wasted) polyamide 6 and polyether polyurethane comprising products can be contaminated with various types of dirt (e.g., with mud, oil, paint or grease).

(Wasted) polyamide 6 and polyether polyurethane comprising products can be mixed with a whole range of other materials, like rocks, glass, metal materials, organic waste and other polymer litter (e.g., polyamide 6,6, polyethylene terephthalate (PET), polypropylene (PP) or polyethylene (PE)).

Preferably, the polyamide 6 and polyether polyurethane comprising material is fragmented into pieces before they are separated in the separation section [B] in step b). This mechanical pre-treatment, i.e., the mechanical comminution or fragmentation of the polyamide 6 and polyether polyurethane comprising material, can be achieved, e.g., by cutting, punching, shredding, milling, grinding and/or chipping. In a preferred embodiment, polyamide 6 and polyether polyurethane comprising material is charged into the separation section [B] of step a) in the form of pieces. Using pieces of polyamide 6 and polyether polyurethane comprising material has the advantage that the pieces can be more easily handled and/or cleaned by washing with a solvent. In a preferred embodiment, the polyamide 6 and polyether polyurethane comprising material is charged into the separation section [B] of step a) in the form of pieces which have on average, along the longest axis of the piece, a length of 1 mm to 100 m, preferably from 3 mm to 1 m and most preferably from 1 cm to 50 cm. The skilled person can easily determine the average length along the longest axis of the employed pieces by first taking a representative sample of the pieces, then measuring the length of the longest axis of each of these pieces (e.g., 50 pieces), and finally calculating the average value of all these individual measurements. The preferred particle dimensions can also be described in terms of average particle weight. Preferably, the pieces of polyamide 6 and polyether polyurethane comprising material have an average particle weight from 0.01 gram to 25 kg, preferably from 0.05 gram to 1 kg and most preferably from 0.1 gram to 100 gram. Experiments have shown that pieces of polyamide 6 and polyether polyurethane comprising material of the aforementioned size or weight dimension are particularly well suited for handling in the process of the invention and/or cleaning by washing with a solvent.

Optionally, prior to the mechanical comminution or fragmentation of the polyamide 6 and polyether polyurethane comprising material, large metal fragments, rocks and other disturbing materials that cause severe wear of the equipment used for the mechanical comminution or fragmentation are removed. Preferably, also foreign materials including, but not limited to, polyethylene, polypropylene and polyamide 6,6 comprising materials are removed prior to the mechanical comminution or fragmentation of the polyamide 6 and polyether polyurethane comprising material. The removal of foreign materials can be done mechanically or manually. The removal of these disturbing materials has the advantage that the maintenance cost of the equipment used for the mechanical comminution or fragmentation can be reduced to a large extent. In addition, the polyamide 6 and polyether polyurethane content of the material obtained after mechanical comminution or fragmentation is higher than without removal of the disturbing materials. In particular the removal of polyamide 6,6 is advantageous because it disturbs the depolymerization of polyamide 6, reduces the recovery yield of ε-caprolactam and disturbs the subsequent purification of the recovered ε-caprolactam.

Optionally, foreign materials are separated from the polyamide 6 and polyether polyurethane comprising material that has been mechanically comminuted or fragmented. To this end, various separation processes are applicable, including, but not limited to, density separation and magnetic separation. In density separation, materials of different densities are placed in a liquid of intermediate density, where the less dense material floats and separates out from the more dense sinking material. In practice, density separation is often done by a series of density separation stages. E.g., in one stage, the high density materials like rocks, sand and metals (including iron and lead) are separated off, while in another stage low density materials, like polyolefins polypropylene and polyethylene, are separated off. Magnetic separation is the process of separating components of mixtures by using magnets to attract magnetic materials. The process that is preferably used for magnetic separation detaches non-magnetic material from magnetic material. The removal of foreign materials in the comminuted or fragmented polyamide 6 and polyether polyurethane comprising product material is advantageous because such materials can disturb the separation of polyamide 6 and polyether polyurethane, the depolymerization of polyamide 6, reduce the recovery yield of ε-caprolactam and/or disturb the subsequent purification of the recovered ε-caprolactam.

Optionally, the polyamide 6 and polyether polyurethane comprising material is cleaned by washing with a solvent, preferably water, prior to being charged to the separation section [B]. Preferably, washing agents ranging in concentrations from 0 to 20 % by weight relative to the solvent are added to the solvent for an improved washing efficiency. NaOH is a preferred washing agent. Even more preferably, an aqueous solution that contains 0 to 10 % by weight NaOH is used in the washing step, still more preferably 0 to 5 % by weight NaOH. The enhanced washing effect of NaOH is most probably caused by enhanced hydrolysis of molecules, including biopolymers and non-biopolymers. Preferably, the washing solvent is heated to further enhance the washing process. In another preferred embodiment, the washing process includes a final rinsing step with (clean) washing solvent without washing agent in order to remove residues of washing agent and dirt present that are adhering to the polyamide 6 and polyether polyurethane comprising material.

The washing is preferably carried out under friction. Different types of industrial friction-applying washing systems are available on the market, like rotary plastic washers and (high speed) friction washers.

Washing of the polyamide 6 and polyether polyurethane comprising material, in particular of mechanically comminuted or fragmented polyamide 6 and polyether polyurethane comprising material, is advantageous because any (adhering) dirt is removed which consequently does not perturb the next steps of the process of the invention.

Optionally, the polyamide 6 and polyether polyurethane comprising material is dried after the cleaning step and prior to being charged to the separation section [B]. This has the advantage that the weight of the cleaned polyamide 6 and polyether polyurethane comprising material is reduced and that the next step is not affected by dilution or by contamination with the washing solvent.

The site where the pre-treatment of the polyamide 6 and polyether polyurethane comprising material is performed and the site where the separation section [B] is located can be the same. However, preferably, one or more of the pre-treatment steps are done at a location different from the location of the separation section [B], e.g., near a plant where the wasted polyamide 6 and polyether polyurethane comprising material is collected and/or at a location that is specialized in pre-treatment of polyamide 6 and polyether polyurethane comprising material. A polyamide 6 rich stream can then be obtained in the separation section [B] from polyamide 6 and polyether polyurethane comprising material that has been pre-treated at various locations.

The site where the separation of the polyamide 6 and polyether polyurethane comprising material in a polyamide 6 rich stream and a polyether polyurethane rich stream is performed and the site where the depolymerization section [C] is located can be the same. However, preferably, separating the polyamide 6 and polyether polyurethane comprising material in a polyamide 6 rich stream and a polyether polyurethane rich stream is done at a location different from the location of the depolymerization section [C], e.g., near a plant where virgin polyether polyurethane is produced and/or at a location that is specialized in handling and/or distilling organic solvents. The site where the pre-treatment of the polyamide 6 and polyether polyurethane comprising material is performed and the site where the depolymerization section [C] is located can be the same. However, preferably, one or more of the pre-treatment steps are done at a location different from the location of the depolymerization section [C], e.g., near a plant where the wasted polyamide 6 and polyether polyurethane comprising material is collected and/or at a location that is specialized in pre-treatment of polyamide 6 and polyether polyurethane comprising material.

The charging step a)

In step a) of the invention, the polyamide 6 and polyether polyurethane comprising material that is optionally pre-treated in pre-treatment section [A], are charged to the separation section [B].

In one embodiment, the polyamide 6 and polyether polyurethane comprising material is mechanically compressed into a smaller volume prior to being charged to the separation section [B]. This has the advantage that less volume is needed for intermediate storage and transport and can also facilitate dosing to the separation section [B].

In another preferred embodiment, the polyamide 6 and polyether polyurethane comprising material is dried before being charged to the separation section [B], in particular after subjecting the polyamide 6 and polyether polyurethane material to a cleaning step. This has the advantage that less or no solvent is introduced into the separation section [B]. Solvent, especially water, introduced in the separation section [B] might negatively influence the separation process (e.g., reduced dissolution rates of polyether polyurethane from the polyamide 6 and polyether polyurethane comprising material in an organic solvent are obtained).

The polyamide 6 and polyether polyurethane comprising material is preferably fed to the dissolution vessel(s) as a solid phase.

The feeding of the polyamide 6 and polyether polyurethane comprising material to the separation section [B] (e.g., feeding to the polyether polyurethane dissolution section) can be realized by continuous or by intermittent dosing of the polyamide 6 and polyether polyurethane comprising material.

The separation step b)

In the separation section [B], the polyamide 6 and polyether polyurethane comprising material is separated to form a polyamide 6 rich stream and a polyether polyurethane rich stream.

Preferably, the process of the invention employs a separation section [B] comprising a dissolution section [α], a washing section [β], a precipitation section [γ], and a solvent distillation section [δ], and comprises the following steps in the separation section:

b.1) charging an organic solvent, and polyamide 6 and polyether polyurethane comprising material to the dissolution section [α];

b.2) dissolving polyether polyurethane from the polyamide 6 and polyether polyurethane comprising material in an organic solvent in the dissolution section [α], so that a polyether polyurethane rich stream comprising organic solvent and dissolved polyether polyurethane, and a stream comprising non-dissolved polyamide 6 are obtained and discharging the obtained streams from the dissolution section [α];

b.3) charging a second solvent and said polyether polyurethane rich stream comprising organic solvent and dissolved polyether polyurethane to the precipitation section [γ] so that polyether polyurethane is precipitated from a mixture comprising organic solvent and second solvent;

b.4) recovering said precipitated polyether polyurethane from said mixture comprising organic solvent and second solvent and discharging said precipitated polyether polyurethane from the precipitation section [γ];

b.5) discharging the mixture comprising organic solvent and second solvent from which precipitated polyether polyurethane has been recovered in step b.4) from the precipitation section [γ] and charging said mixture to the solvent distillation section [δ];

b.6) charging a third solvent and the stream comprising non-dissolved polyamide 6 to the washing section [β];

b.7) washing the stream comprising non-dissolved polyamide 6 with the third solvent in the washing section [β], so that a polyamide 6 rich stream and a mixture comprising organic solvent and third solvent are obtained;

b.8) discharging said polyamide 6 rich stream from the washing section [β];

b.9) discharging the mixture comprising organic solvent and third solvent that is obtained in step b.7) from the washing section [β] and charging said mixture to the solvent distillation section [δ];

b.10) separating organic solvent from the second solvent and the third solvent by distillation in the solvent distillation section [δ] and discharging the second solvent, the third solvent, and the separated organic solvent from the solvent distillation section [δ].

Optionally the second solvent (see step b.3)) can be partly or completely the mixture comprising organic solvent and third solvent that is obtained in step b.7) from the washing section [β].

Preferably, the separation section [B] employed in the process of the invention comprises a dissolution section [α], a washing section [β], a precipitation section [γ], and a solvent distillation section [δ]. Such a separation section [B] is particularly suitable to perform the separation of the polyamide 6 and polyether polyurethane comprising material in a polyamide 6 rich stream and a polyether polyurethane rich stream according to the process of the invention. Surprisingly, the separation of the polyamide 6 and polyether polyurethane comprising material in a polyamide 6 rich stream and a polyether polyurethane rich stream is particularly effective when performing the process steps b.1) to b.10) in the separation section [B] comprising a dissolution section [α], a washing section [β], a precipitation section [γ], and a solvent distillation section [δ]. Steps b.1) to b.10) taking place in the preferred separation section [B] are further explained in the following. Steps b.1) to b.10) can replace process steps a) and b) in the process of the invention. Step b.1) thereby corresponds to step a).

A "rich stream" as used herein is a stream which contains the enriched component in a greater amount than another stream obtained in the same process step. The polyamide 6 rich stream therefore contains more polyamide 6 than the polyether polyurethane rich stream. In contrast, the polyether polyurethane rich stream contains more polyether polyurethane than the polyamide 6 rich stream.

In particular, the polyamide 6 rich stream has a weight to weight ratio of polyamide 6 to polyether polyurethane that is higher than the weight to weight ratio of polyamide 6 to polyether polyurethane of the polyamide 6 and polyether polyurethane comprising material.

Preferably, the polyamide 6 rich stream has a weight to weight ratio of polyether polyurethane to polyamide 6 that is at least two times lower than the weight to weight ratio of polyether polyurethane to polyamide 6 of the polyamide 6 and polyether polyurethane comprising material, more preferably at least three times lower.

The polyether polyurethane rich stream has a weight to weight ratio of polyether polyurethane to polyamide 6 that is higher than the weight to weight ratio of polyether polyurethane to polyamide 6 of the polyamide 6 and polyether polyurethane comprising material.

Preferably, the polyether polyurethane rich stream has a weight to weight ratio of polyether polyurethane to polyamide 6 that is at least two times higher than the weight to weight ratio of polyether polyurethane to polyamide 6 of the polyamide 6 and polyether polyurethane comprising material, more preferably at least three times higher.

*The charging to separation section step (step b. 1)):*

In separation section [B], an organic solvent, and polyamide 6 and polyether polyurethane comprising material is charged to the dissolution section [α].

The charging of the polyamide 6 and polyether polyurethane comprising material to the dissolution section [α] is the charging of the polyamide 6 and polyether polyurethane comprising material that is optionally pre-treated in pre-treatment section [A] as mentioned in step a) of the invention.

The organic solvent that is charged to the dissolution section [α] can be any organic solvent in which polyether polyurethane can be dissolved. Preferably, the organic solvent combines a (very) good solubility for polyether polyurethane and a (very) poor solubility for polyamide 6. According to another preferred embodiment, the organic solvent is selected from N,N-dimethylformamide (DMF, $(CH_3)_2NC(=O)H$), dimethylacetamide (DMAc, $CH_3C(=O)N(CH_3)_2$), 1,4-dioxane (dioxane, $C_4H_8O_2$), N-alkyl-2-pyrrolidones such as N-methyl-2-pyrrolidone (NMP, $C_5H_9NO$), tetrahydrofuran (THF; a.k.a. oxolane, $C_4H_8O$) and combinations thereof. Even more preferably, the organic solvent is selected from N,N-dimethylformamide and dimethylacetamide. Most preferably, the organic solvent is dimethylacetamide.

The organic solvent that is charged to the dissolution section [α] can be a separated organic solvent (i.e., recycled organic solvent or an organic solvent from inside the process, that has been recovered in a solvent distillation section), fresh organic solvent (i.e., solvent from outside the process) or a combination of separated organic solvent and fresh organic solvent.

The organic solvent that is charged to the dissolution section [α] preferably has a low water content. Even more preferably, the organic solvent is dried (preferably by distillation or applying a drying agent, like an inorganic salt such as $Na_2SO_4$, a zeolite, silica and alumina) before being charged to the dissolution section [α]. Drying is not necessarily to completion. Typically, less than 1 wt.%, preferably, less than 0.1 wt.%, more preferably, less than 0.02 wt.% water remains after drying the organic solvent.

*The dissolving step (step b.2)):*

In dissolution section [α] polyether polyurethane is dissolved from the polyamide 6 and polyether polyurethane comprising material in the organic solvent that was charged to the dissolution section [α] in step b.1). Hereby a polyether polyurethane rich stream comprising organic solvent and dissolved polyether polyurethane, and a stream comprising non-dissolved polyamide 6 are obtained that are discharged from dissolution section [α].

Preferably, dissolution of polyether polyurethane will be complete in 0.1 hour to 24 hours, more preferably in 0.5 hour to 6 hours. Complete means that the dissolution has reached a plateau with no substantial further dissolution occurring at later timepoints.

The temperature in the dissolution section [α] can vary and is preferably below 120 °C, more preferably below 110 °C, even more preferably below 100 °C and most preferably below 80 °C. Preferably, the temperature in the dissolution section [α] may range from 0 °C to 100 °C, more preferably from 10 °C to 90 °C, even more preferably from 10 °C to 80 °C, and most preferably from 20 °C to 75 °C. Experiments have shown that these temperature ranges result in a particularly complete dissolution of polyether polyurethane. This has the advantage that the polyether polyurethane present in the polyamide 6 and polyether polyurethane comprising material can be dissolved. Another advantage of these temperatures is that degradation of polyether polyurethane is minimized or even completely prevented.

The amount of solvent (expressed in tons) in the dissolution section [α] can vary and might range from 0.5 to 100 times, preferably from 1 to 60 times, more preferably from 2 to 20 times, most preferably from 3 to 10 times the amount of polyether polyurethane (expressed in tons) in the dissolution section [α]. A low amount of solvent reduces the dissolution rate of polyether polyurethane and might even result in incomplete dissolution of the polyether polyurethane. A large amount of solvent gives a high dissolution rate of polyether polyurethane, however requires more energy and costs for the recovery of the used organic solvent. The skilled person is able to determine the optimal ratio of solvent to polyether polyurethane or nylon 6 and polyether polyurethane comprising material by way of routine tests.

The dissolution section [α] might comprise one or more dissolution vessels that are operated in series or in parallel. Preferably, these vessels are equipped with additional equipment (e.g., stirrers) to enhance the friction, which reduces the time of dissolution.

*The precipitation step (step b.3)):*

The polyether polyurethane rich stream comprising organic solvent and dissolved polyether polyurethane that was discharged from the dissolving section [α] and a second solvent are charged to the precipitation section [γ] so that polyether polyurethane is precipitated from a mixture comprising organic solvent and second solvent.

Preferably, the second solvent is a non-solvent (a.k.a. anti-solvent) for polyether polyurethane. The addition of this second solvent reduces the solvent power for the polyether polyurethane dissolved in the solution, which leads to precipitation of particles of precipitated polyether polyurethane. The second solvent can be any solvent. Preferably, the second solvent is water. Water as second solvent has the advantage that it is an environmentally benign solvent.

In a preferred embodiment, the second solvent is the mixture comprising organic solvent and third solvent that is obtained in step b.7) from the washing section [β]. In another preferred embodiment, the second solvent consists of part of or is the mixture comprising organic solvent and third solvent that is obtained in step b.7) from the washing section [β]. "Part of the mixture" as used herein is defined as any fraction between 0 and 100 wt.%, preferably, between 10 and 100 wt.%, more preferably, between 25 and 100 wt.%, even more preferably, between 75 to 100 wt.%. This has the advantage that less fresh solvent is used for the precipitation of polyether polyurethane. Another advantage is that less solvent needs to be distilled in solvent distillation section [δ], which results in lower energy costs, smaller sized equipment, reduced investment costs and a more environmentally benign process.

The temperature in the precipitation section [γ] can vary and might range from 0 °C to 150 °C, preferably from 10 °C to 100 °C, more preferably from 15 °C to 80 °C and most preferably from 20 °C to 60 °C. Experiments have shown that these temperature ranges result in particularly complete precipitation of the dissolved polyether polyurethane.

The amount of second solvent charged to the precipitation section [γ] can vary and might range from 0.1 wt.% to 500 wt.%, preferably from 0.2 wt.% to 100 wt.%, more preferably from 0.5 wt.% to 50 wt.% and most preferably from 1 wt.% to 25 wt.% compared to the organic solvent charged to the precipitation section [γ].

A low amount of second solvent reduces the degree of precipitation of polyether polyurethane. A large amount of second solvent complicates the recovery of precipitated polyether polyurethane and the organic solvent. A large amount of second solvent also requires more energy and costs for the recovery of precipitated polyether polyurethane and the organic solvent. The skilled person will be able to find the optimal ratio of second solvent to polyether polyurethane rich stream for the specific solvent and equipment used by carrying out simple precipitation test trials.

*The polyether polyurethane recovery step (step b.4):*

In the polyether polyurethane recovery step precipitated polyether polyurethane is recovered from the mixture comprising organic solvent and second solvent and discharged from precipitation section [γ]. There are several suitable methods to recover a precipitate, including, but not limited to, filtration, centrifugation and decantation. Preferably, filtration is used to recover polyether polyurethane. For filtration, the solution containing the precipitate is charged to a filter, whereby the precipitate is expected to remain on the filter, while the liquid passes through it. When using centrifugation as recovery method, the solution containing the precipitate is rapidly rotated so that the solid precipitate settles (assuming that the solid precipitate has a higher density than the liquid). The compacted precipitate can also be obtained by pouring off the liquid. In decantation, the liquid layer is poured or suctioned away from the precipitate.

Optionally, the recovered precipitate is washed with a solvent before being discharged from precipitation section [γ]. Preferably, the solvent that is used to wash the recovered precipitate is either the organic solvent of step b.1) or second solvent.

Optionally, the recovered precipitate that is optionally washed with a solvent is dried before being discharged from precipitation section [γ]. In a preferred embodiment, the precipitated polyether polyurethane that is discharged from the precipitation section [γ] in step b.4) is re-used, optionally in combination with fresh polyether polyurethane, in the production of textiles.

*The discharging solvent mixture step (step b.5)):*

The mixture comprising organic solvent and second solvent from which precipitated polyether polyurethane has been recovered in step b.4) is discharged from precipitation section [γ] and charged to solvent distillation section [δ]. Optionally, solvent resulting from washing of the recovered precipitate is also discharged from precipitation section [γ] and charged to solvent distillation section [δ].

*The charging to washing step (step b.6)):*

In separation section [B], a third liquid solvent, and the stream comprising non-dissolved polyamide 6 are charged to the washing section [β]. The stream comprising non-dissolved polyamide 6 that is charged to the washing section [β] can comprise next to non-dissolved polyamide 6 also organic solvent. The purpose of the washing section [β] is to recover organic solvent that is present in the stream comprising non-dissolved polyamide 6, whereby a polyamide 6 rich stream is obtained that contains less organic solvent than the stream comprising non-dissolved polyamide 6.
Preferably, the third solvent has a good solubility for the organic solvent and a poor solubility for polyamide 6. The third solvent can be any solvent. Preferably, the second solvent is water. Water as third solvent has the advantage that it is an environmentally benign solvent.
The amount of third solvent charged to the washing section [β] can vary and might range from 10 wt.% to 1000 wt.%, preferably from 20 wt.% to 500 wt.%, more preferably from 50 wt.% to 400 wt.% and most preferably from 100 wt.% to 250 wt.% compared to the stream comprising non-dissolved nylon 6 that is charged to the washing section [β].

*The washing step (step b.7)):*

In washing section [β], the stream comprising non-dissolved polyamide 6 is washed with the third solvent so that a polyamide 6 rich stream and mixture comprising organic solvent and third solvent are obtained.
The temperature in the washing section [β] can vary and might range from 0 °C to 150 °C, preferably from 10 °C to 100 °C, more preferably from 15 °C to 80 °C and most preferably from 20 °C to 60 °C. Experiments have shown that these temperature ranges resulted in a particularly complete recovery of the organic solvent from the stream comprising non-dissolved polyamide 6.
The skilled person can determine by routine experimentation the amount of third solvent and the optimal temperature necessary for efficient washing of the stream comprising non-dissolved polyamide 6.
The washing of the stream comprising non-dissolved polyamide 6 with a third solvent can be performed in various devices, all of which are known to the skilled person.

*The discharging of polyamide 6 rich stream step (step b.8)):*

From the washing section [β] the polyamide 6 rich stream is discharged. This polyamide 6 rich stream comprises third solvent and optionally organic solvent. Preferably, the organic solvent content of the polyamide 6 rich stream is less than 25 wt.%, preferably less than 10 wt.%, more preferably less than 2 wt.% and most preferably less than 0.2 wt.% relative to the total weight of the polyamide 6 rich stream. Optionally, the polyamide 6 rich stream is dried prior to being discharged.
Preferably, the polyamide 6 content of the resulting polyamide 6 rich stream from washing section [β] is at least 85 %, more preferably at least 90 % and most preferably at least 95 % by weight on dry-weight basis.
Preferably, the polyamide 6 content of the resulting polyamide 6 rich stream from separation section [B] is at least 85 %, more preferably at least 90 %, even more preferably at least 95 % and most preferably at least 98 % by weight on dry-weight basis.

Determination of the polyamide 6 content is a routine activity and can be performed by various methods, all of which are known to the skilled person. Preferably, the polyamide 6 content of the polyamide 6 rich stream is determined by Thermogravimetric analysis (TGA) and/or by DSC (e.g., by method ISO 11357-3).
As used herein, the expression "dry-weight basis" refers to the content of an ingredient expressed as a percentage of the total dry-weight of the composition, wherein "dry-weight" is the weight of a substance after removing the water and/or other liquids from the substance by drying until weight constancy.

*The discharging of mixture comprising organic solvent and third solvent step (step b.9)):*

From the washing section [β] the mixture comprising organic solvent and third solvent is discharged. Generally, the organic solvent and/or the third solvent are valuable compounds and recovery and reuse has great economic and environmental advantages. The mixture comprising organic solvent and third solvent is charged to solvent distillation section [δ]. Optionally, the mixture comprising organic solvent and third solvent is first charged to another section before being charged to solvent distillation section [δ]. In a preferred embodiment, the mixture comprising organic solvent and third solvent is first partly or completely charged to the precipitation section [γ].

*The solvent separation step (step b. 10)):*

In solvent distillation section [δ], the mixture comprising organic solvent and second solvent from which precipitated polyether polyurethane has been recovered and the mixture comprising organic solvent and third solvent that was discharged from washing section [β] are separated by distillation. Hereafter, the mixture comprising organic solvent and second solvent from which precipitated polyether polyurethane has been recovered and the mixture comprising organic solvent and third solvent that was discharged from washing section [β] are called combined feed to solvent distillation section [δ].

From the solvent distillation section [δ] the separated organic solvent, the second solvent and the third solvent, and residues are discharged. In a preferred embodiment, the separated organic solvent that is discharged from the solvent distillation section [δ] in step b.10) is charged to the dissolution section [α] in step b.1). In another preferred embodiment, the second solvent and the third solvent that are discharged from the solvent distillation section [δ], optionally after separation from each other, are re-used in the precipitation section [γ] and/or in the washing section [β].

The separation by distillation is used to separate liquids from non-volatile solids and to separate liquids having different boiling points. Distillation is a routine activity and can be performed in various devices, all of which are known to the skilled person.

The mixture comprising organic solvent and second solvent from which precipitated polyether polyurethane has been recovered, optionally also contains (dissolved) polyether polyurethane, (dissolved) polyamide 6, and degradation products of the organic solvent and/or the second solvent. The mixture comprising organic solvent and third solvent optionally also contains (dissolved) polyether polyurethane, (dissolved) polyamide 6, and degradation products of the organic solvent and/or the third solvent.

In general, the separation of three different liquids by distillation is more complex, requires more energy and requires more equipment than the separation of two different liquids by distillation. Thus, there is a desire to limit the number of different solvents in the process of the invention to two. In a preferred embodiment of the invention, the second solvent and the third solvent are the same solvent. In a more preferred embodiment, the second solvent and the third solvent are water. The use of water as a solvent is advantageous because it is cheap, abundantly available, non-explosive and environmentally benign.

In a preferred embodiment of the invention, the organic solvent is selected from N,N-dimethylformamide (DMF, $(CH_3)_2NC(=O)H$), N,N-dimethylacetamide (DMAc, $CH_3C(=O)N(CH_3)_2$), 1,4-dioxane (dioxane, $C_4H_8O_2$), N-alkyl-2-pyrrolidones such as N-methyl-2-pyrrolidone (NMP, $C_5H_9NO$), tetrahydrofuran (THF; a.k.a. oxolane, $C_4H_8O$). In a more preferred embodiment, the organic solvent is N,N-dimethylacetamide (DMAc, $CH_3C(=O)N(CH_3)_2$).

In another preferred embodiment, the organic solvent is selected from N,N-dimethylformamide (DMF, $(CH_3)_2NC(=O)H$), N,N-dimethylacetamide (DMAc, $CH_3C(=O)N(CH_3)_2$), 1,4-dioxane (dioxane, $C_4H_8O_2$), N-alkyl-2-pyrrolidones such as N-methyl-2-pyrrolidone (NMP, $C_5H_9NO$), tetrahydrofuran (THF; a.k.a. oxolane, $C_4H_8O$) and the second solvent and the third solvent are both water. In a more preferred embodiment of the invention, the organic solvent is N,N-dimethylacetamide (DMAc, $CH_3C(=O)N(CH_3)_2$), and the second solvent and the third solvent are both water. The boiling point of N,N-dimethylacetamide (DMAc) at atmospheric pressure is ca. 165 °C. The boiling point of water at atmospheric pressure is 100 °C.

When only N,N-dimethylacetamide (DMAc) and water are present in the combined feed to solvent distillation section [δ], the volatility difference between the two is large and separation by distillation will be rather easy. In such situations continuous separation of the combined feed to solvent distillation section [δ] might be realized in a single distillation column, that is operated in a continuous mode. In such a distillation column the water is distilled from the top of the column in a gaseous state, and N,N-dimethylacetamide (DMAc) is withdrawn from the bottom of the column in a liquid state. When besides N,N-dimethylacetamide (DMAc) and water also non-volatile compounds (like polyether polyurethane and polyamide 6) are present in the combined feed to solvent distillation section [δ], the lay-out of the solvent distillation required for the continuous separation into N,N-dimethylacetamide (DMAc), water and non-volatile compounds is more extended. A straightforward lay-out for this type of separation by distillation comprises two distillation columns that are operated in series. The feed (in liquid state) that consists of N,N-dimethylacetamide (DMAc), water and the non-volatile compounds is charged to the first distillation column. The water is distilled from the top of the first distillation column in a gaseous state, and a mixture of N,N-dimethylacetamide (DMAc) and the non-volatile compounds are withdrawn from the bottom of the first distillation column (in a liquid state, or as a slurry). The bottom flow of the first column is charged to the second distillation column. The N,N-dimethylacetamide (DMAc) is distilled from the top of the second distillation column in a gaseous state, and the non-volatile compounds are withdrawn from the bottom of the second distillation column. In practice a mixture of N,N-dimethylacetamide (DMAc) and non-volatile compounds might be withdrawn from the bottom of the second distillation column in order to reduce the risk of clogging of the second distillation column. A squeezing (column) unit, that recovers N,N-dimethylacetamide (DMAc) from the bottom flow of the second distillation column, might be added to enhance the overall recovery of N,N-dimethylacetamide (DMAc).

As an alternative for the separation by distillation of two distillation columns that are operated in series, a single distillation column with side withdrawal that is operated in a continuous mode can be chosen. In such a distillation column, the water is distilled from the top of the distillation column in a gaseous state, the N,N-dimethylacetamide (DMAc) is discharged as a liquid side stream and the non-volatile compounds are withdrawn from the bottom of the second distillation column. Optionally, the bottom flow is charged to a squeezing (column) unit.

As an alternative for charging the feed to the distillation column in liquid state, the charging to the first distillation column can be done in the gaseous state. In such a case the combined feed to solvent distillation section [δ] with the exception of the non-volatile compounds is evaporated in a feed evaporator prior to charging to the first distillation column. The non-volatile compounds are discharged from the feed evaporator. This alternative has the advantage that less fouling occurs in the distillation columns. Optionally, the squeezing column can be combined with the feed evaporator. Generally, N,N-dimethylacetamide (DMAc) comprising process flows contain certain amounts of acetic acid and dimethylamine, due to, e.g., the degradation of N,N-dimethylacetamide (DMAc). This is also the case for the combined feed to solvent distillation section [δ].

The boiling point of pure dimethylamine at atmospheric pressure is ca. 7 °C. Dimethylamine dissolves very well in water.

The boiling point of pure acetic acid at atmospheric pressure is ca. 118 °C.

However, acetic acid and N,N-dimethylacetamide (DMAc) form a high-boiling azeotrope (with a composition of ca. 21 wt.% acetic acid and ca. 79 wt.% N,N-dimethylacetamide at atmospheric pressure) and the atmospheric boiling point is ca. 171 °C, which is just somewhat higher than the atmospheric boiling point of N,N-dimethylacetamide. A high-boiling azeotrope (or constant boiling point mixture) is a mixture of two liquids that has a boiling point that is higher than the boiling points of the two individual liquids (in pure form).

If the pressure is reduced, the azeotropic point of the acetic acid and N,N-dimethylacetamide mixture shifts to higher concentrations of the low boiling component (which is acetic acid).

At 6.7 kPa a high-boiling azeotrope with a composition of ca. 30 wt.% acetic acid and ca. 70 wt.% N,N-dimethylacetamide is formed with a boiling point of ca. 93 °C.

At 1.3 kPa a high-boiling azeotrope with a composition of ca. 42 wt.% acetic acid and ca. 58 wt.% N,N-dimethylacetamide is formed with a boiling point of ca. 75 °C. Preferably, the degradation products are removed from the solvents that are re-used in the process. This has the advantage that an accumulation of degradation products is prevented. In a preferred embodiment, degradation products of the organic solvent are removed prior to charging the separated organic solvent that is discharged from the solvent distillation section [δ] in step b.10) to the dissolution section [α] in step b.1). However, N,N-dimethylacetamide (DMAc) and acetic acid cannot be separated by simple distillation due to the formation of the maximum azeotrope.

To the man skilled in the art, several techniques are known to break an azeotrope in distillation.

One group of techniques is based on the addition of another compound which changes the molecular interactions and eliminates the N,N-dimethylacetamide-acetic acid maximum azeotrope. Chlorobenzene, toluene, ethylbenzene and xylene are examples of compounds that break this maximum azeotrope. Addition of one of these compounds to a mixture of N,N-dimethylacetamide and acetic acid results in the formation of a new azeotrope of that compound and acetic acid that can be removed by evaporation. Subsequently, the resulting mixture of that compound and acetic acid is separated.

Another group of techniques, often called pressure-swing distillations, is based on the fact that the maximum azeotrope is pressure dependent. In such systems, two distillation columns are operated at different pressure levels. The feed mixture of N,N-dimethylacetamide and acetic acid is charged to a first distillation column that is operated at a certain pressure. The bottom flow of this first distillation column is charged to the second distillation column. The bottom flow of the second distillation column is charged to the first distillation column. As top products of the distillation columns high purity N,N-dimethylacetamide and acetic acid are obtained. A major advantage of pressure-swing distillations is the absence of foreign compounds.

Finally, there is a group of techniques that converts acetic acid into another component (e.g., salt formation due to addition of caustic) or that selectively adsorbs acetic acid from the mixture of N,N-dimethylacetamide and acetic acid.

When besides N,N-dimethylacetamide (DMAc), water and non-volatile compounds (like polyether polyurethane and polyamide 6), also acetic acid and dimethylamine are present in the combined feed to solvent distillation section [δ], the lay-out of the solvent distillation required for the continuous separation into N,N-dimethylacetamide (DMAc), water, non-volatile compounds, acetic acid and dimethylamine is even more extended.

The optimal lay-out is very much dependent on both the composition and the volume flow of the combined feed to solvent distillation section [δ]. The lay-out might contain a combination of various separation solutions that were described before.

In case the fraction of acetic acid in the combined feed to solvent distillation section [δ] is rather low, the solvent distillation section [δ] might comprise two distillation columns that are operated in series, followed by a neutralizer-evaporation step. The feed that consists of N,N-dimethylacetamide (DMAc), water, non-volatile compounds, acetic acid and dimethylamine is charged to the first distillation column. The water and dimethylamine are distilled from the top of the first distillation column in a gaseous state, and a mixture of N,N-dimethylacetamide (DMAc), acetic acid and the non-volatile compounds are withdrawn from the bottom of the first distillation column (in a liquid state, or as a slurry). The water vapors from the top of the first distillation column are condensed. The resulting liquid phase contains water and dissolved dimethylamine. In a dimethylamine stripping unit the dimethylamine can be removed from this liquid phase, whereby water is obtained that is (almost) free of dimethylamine. The bottom flow of the first column is charged to the second distillation column. The N,N-dimethylacetamide (DMAc) is distilled from the top of the second distillation column in a gaseous state, and a mixture of N,N-dimethylacetamide (DMAc), acetic acid and the non-volatile compounds are withdrawn from the bottom of the second distillation column. The bottom flow of the second column is charged to the neutralizer-evaporation unit. To the neutralizer-evaporation unit a base, e.g., aqueous NaOH, is charged to neutralize the acetic acid. Due to evaporation, gaseous N,N-dimethylacetamide (DMAC) is discharged from the neutralizer-evaporation unit. If present also water is evaporated. A mixture of neutralized acetic acid (acetic acid containing salt) and non-volatile compounds is discharged from the bottom of the neutralizer-evaporation unit. In practice a mixture of N,N-dimethylacetamide (DMAc), neutralized acetic acid (acetic acid containing salt) and non-volatile compounds might be withdrawn from the bottom of the neutralizer-evaporation unit in order to reduce risk of clogging of the neutralizer-evaporation unit. The N,N-dimethylacetamide (DMAc) that is recovered by distillation is discharged from solvent distillation section [δ] and charged to dissolution section [α].

Preferably, water that is recovered in solvent distillation section [δ], optionally after removal of dimethylamine, is re-used in the process of the invention. Preferably, water is used as second solvent and/or as third solvent.

The charging step c.1)

In step c.1) of the invention, the resulting polyamide 6 rich stream is charged to the depolymerization section [C] wherein the polyamide 6 content of the polyamide 6 rich stream is at least 85 %, preferably at least 90 % by weight on dry-weight basis. This has the advantage that the polyamide 6 rich stream that is charged to the depolymerization section [C] is less contaminated with foreign materials, which improves the yield and the purity of the ε-caprolactam that is produced in the plant of the invention configured to carry out the process of the invention. Other advantages are that a polyamide 6 rich stream will require less depolymerization agent, will produce less waste and will require less energy in the depolymerization section [C]. And finally, a polyamide 6 rich stream will cause less operational problems, like fouling and clogging of equipment. The depolymerization section [C] comprises one or more depolymerization reactors that are operated in series and/or in parallel.

Optionally, the polyamide 6 rich stream is mechanically compressed into a smaller volume prior to being charged to the depolymerization section [C]. This has the advantage that less volume is needed for intermediate storage and transport and can also facilitate dosing to the depolymerization section [C].

Optionally, the polyamide 6 rich stream is compressed into particles with an increased density prior to being charged to the depolymerization section [C]. This can be achieved, e.g., by mechanical compaction or by extrusion of melted material through a die followed by cooling and cutting it to size. Compression of the polyamide 6 rich stream into particles has the advantage of an increased bulk density, which reduces the costs of intermediate storage and transportation. Apart from density increase, pelletization also offers other benefits, such as a homogeneous shape and structure that is advantageous for (automated) feeding into the depolymerization section [C].

Optionally, polyamide 6 rich stream is charged to a smelter (e.g., an extruder). In the smelter the polyamide 6 rich stream is melted. Preferably, the resulting polymer melt is filtered. This has the advantage that solid impurities are removed. The melted and optionally filtered polymer melt is cooled and then fed to a pelletizer. The pelletizer cuts the product into pellets. These pellets are charged to the depolymerization section [C].

The dimensions and shape of the pellets (also often called granules) can be chosen within wide limits. In general, pellets are cylindrical in shape (originating from thin strands that are chopped into pieces). Other shapes like (non-perfect) spheres, however, are also possible. The dimensions of the pellets can be chosen within wide limits. Pellets can have a diameter that ranges from 1 to 10 mm, preferably from 2 to 5 mm, more preferably from 3 to 5 mm. In a preferred embodiment, pellets have a length that ranges from 1 to 50 mm, preferably from 2 to 25 mm and more preferably from 5 to 15 mm.

Optionally, the polyamide 6 rich stream that is discharged from separation section [B] is dried prior to being charged to depolymerization section [C]. This has the advantage that less or no solvent is introduced in the depolymerization section [C]. Solvent introduced in the depolymerization section [C] can negatively influence the depolymerization process (e.g., reduced depolymerization reaction rates, higher consumption of catalyst, higher energy consumption, and the vapor stream comprising $\varepsilon$-caprolactam and water that is obtained in the depolymerization section [C] can contain more impurities).

The polyamide 6 rich stream is preferably fed to the depolymerization reactor(s) as a solid phase or as a melt. Preferably, the polyamide 6 rich stream is charged as a melt. Feeding as a melt may be achieved by using an extruder, gear pump, or other means known by the skilled person.

The feeding of the polyamide 6 rich stream to the depolymerization reactor(s) may be realized by continuous or by intermittent dosing of the polyamide 6 rich stream.

The depolymerization step c.2)

In the depolymerization section [C], the polyamide 6 rich stream is depolymerized to form $\varepsilon$-caprolactam. The formed $\varepsilon$-caprolactam is discharged from the depolymerization section [C] as an $\varepsilon$-caprolactam comprising stream.

The depolymerization of the polyamide 6 rich stream is achieved by increasing the temperature of the polyamide 6 rich stream to a temperature of at least 180 °C but not higher than 400 °C. The preferred temperature range for the depolymerization reaction is from 200 °C to 350 °C, more preferably from 220 °C to 340 °C, and most preferably from 240 °C to 325 °C. Experiments have shown that in these temperature ranges the depolymerization is particularly effective and less side reactions of polyamide 6 and $\varepsilon$-caprolactam and reactions of impurities occur.

Generally, the rate of $\varepsilon$-caprolactam formation increases at elevated temperatures. Temperatures lower than 400 °C are preferred since at temperatures above 400 °C side reactions of polyamide 6 and $\varepsilon$-caprolactam and reactions of impurities occur more frequently which will result in formation of a more diverse set of impurities. Part of these impurities will end-up in the $\varepsilon$-caprolactam comprising product stream that is discharged from the depolymerization section [C]. In a preferred embodiment, the depolymerization of the polyamide 6 rich stream is conducted at temperatures ranging of from 220 °C to 340 °C or 240 °C to 325 °C. Experiments have shown that this temperature range allows production of particularly pure $\varepsilon$-caprolactam.

The pressure in the depolymerization section [C] can vary and might range from 1 kPa to 100 MPa, preferably from 10 kPa to 5 MPa, more preferably from 25 kPa to 2 MPa, most preferably from 50 kPa to 1 MPa. This pressure range allows production of particularly pure $\varepsilon$-caprolactam.

The depolymerization of the polyamide 6 rich stream can be achieved in the presence or in the absence of a solvent. Preferably, the depolymerization of the polyamide 6 rich stream is achieved in the presence of water as solvent. In this case the water is preferably in the form of steam, in particular superheated steam.

Preferably, depolymerization will be complete in 0.1 hour to 24 hours, more preferably in 0.5 hour to 6 hours.

Feeding the water as steam to the depolymerization reactor allows, optionally without further heating, to obtain a vapor stream comprising $\varepsilon$-caprolactam and water. The weight to weight ratio of $\varepsilon$-caprolactam to water in this vapor stream can be adjusted by modifying the amount of steam that is fed to the polyamide 6 rich stream in the depolymerization section [C]. In a preferred embodiment, the depolymerization in step c.2) is performed in the presence of water so that the $\varepsilon$-caprolactam comprising stream is a vapor stream comprising $\varepsilon$-caprolactam and water in a weight to weight ratio of from 1:1 to 1:50, preferably from 1:2 to 1:15, more preferably from 1:2 to 1:10 and most preferably from 1:3 to 1:8. Within these ranges, a particularly economically efficient process can be conducted.

Preferably, the $\varepsilon$-caprolactam in the vapor stream comprising $\varepsilon$-caprolactam and water has a pressure of 0.1 kPa to 1 MPa, more preferably of 0.3 kPa to 0.5 MPa and most preferably of 1 kPa to 0.1 MPa.

During the depolymerization reaction, decomposition products may be formed including linear oligomers of $\varepsilon$-caprolactam and cyclic oligomers of $\varepsilon$-caprolactam. In addition, the feed stream of the polyamide 6 rich stream may also contain other components, i.e., impurities such as non-polyamide 6 compounds and residues of solvents(s) applied in the pre-treatment section and/or separation section that remain stable, react or decompose under the depolymerization conditions. Thus, in case water is used as solvent in the depolymerization section [C], the vapor stream which is removed from the depolymerization section [C] does not only comprise water and $\varepsilon$-caprolactam, but also impurities.

Preferably, superheated steam with a temperature between 100 °C and 600 °C is charged to the depolymerization reactor(s). Preferably, the superheated steam that is charged to the depolymerization reactor(s) has a temperature of at least the melting temperature of polyamide 6. Preferably, the energy content of the superheated steam that is charged to the depolymerization reactor(s) is sufficiently high so that no other heat input is needed for performing the depolymerization reaction and evaporating the formed $\varepsilon$-caprolactam. In another preferred embodiment, the depolymerization section [C] is charged with superheated steam having a temperature ranging of from 220 °C to 575 °C. In an even more preferred embodiment, the depolymerization section [C] is charged with superheated steam having a temperature ranging of from 275 °C to 500 °C.

Generally, the mass of the vapor stream, which is removed from the depolymerization section [C], is less than the mass of the total feed to the depolymerization section. The total feed to the depolymerization section [C] comprises a polyamide 6 rich stream and optionally solvent, catalyst, additional agents and/or depolymerizing agents. Thus, without any additional measures, there will be an accumulation of material (often called 'residual material') in the depolymerization section [C]. Preferably, another stream is discharged from the depolymerization section [C]. This has the advantage that the accumulation of material in the depolymerization section [C] is reduced or avoided. The additional stream that is discharged can comprise impurities present in the polyamide 6 rich stream, non-depolymerized polyamide, non-evaporated ε-caprolactam, catalyst(s) and compounds that were formed under the depolymerization conditions, like mono-, di- and/or triammonium phosphate, in case phosphoric acid is used as depolymerization catalyst. In a preferred embodiment, a stream comprising mono-, di- and/or triammonium phosphate is discharged from the depolymerization section [C]. Even more preferably, this stream which is intermittently or continuously discharged from the depolymerization section [C] comprises mono-, di- and/or triammonium phosphate in a weight fraction of 0.01 to 50 % by weight, preferably from 0.1 to 25 % by weight, more preferably from 0.5 to 10 % by weight, most preferably from 0.5 to 5 % by weight.

The depolymerization of the polyamide 6 rich stream in the presence of steam can be performed with the presence of additional depolymerizing agents, such as ammonia. The concentration of ammonia in the depolymerization section [C] can vary. Thus, in case ammonia is present in the depolymerization section [C], then the vapor stream which is removed from the depolymerization section [C] does not only comprise ε-caprolactam and impurities, but may also comprise ammonia.

Most preferably, the depolymerization is carried out in the presence of a catalyst. Preferably, the used catalyst is a (Lewis or Brønsted) acid or base. The acid catalyst can in particular be selected from the group consisting of orthophosphoric acid, p-toluenesulfonic acid, boric acid, sulfuric acid, organic acid, organic sulfonic acid including xylene sulfonic acid, 4-sulfoisophthalic acid and other sulfonated aromatic hydrocarbons, solid acid, salts of the aforementioned acids, $Al_2O_3$ and $SiO_2$, and combinations thereof. The base catalyst can, e.g., be selected from the group consisting of alkali hydroxide, alkali salt, alkaline earth hydroxide and alkaline earth salts, organic bases and solid bases, and combinations thereof. Preferably, orthophosphoric acid, boric acid, organic acid, alkali hydroxides and alkali salts are used as catalysts. More preferably, orthophosphoric acid, sodium phosphate, potassium phosphate, sodium hydroxide, potassium hydroxide, sodium carbonate, potassium carbonate, sodium hydrogen carbonate and potassium hydrogen carbonate are used as catalysts. Still more preferably, orthophosphoric acid, p-toluenesulfonic acid, boric acid and sodium hydroxide are used as catalysts. In one particularly preferred embodiment, orthophosphoric acid is used as catalyst for the depolymerization, in another, p-toluenesulfonic acid is used.

In another preferred embodiment, however, no catalyst is used for the depolymerization of the polyamide 6 rich stream. This has the advantage of lower costs (both for the catalyst and the disposal of catalyst waste). However, higher temperatures (and pressures) are usually required.

The advantage of using a catalyst (and especially of orthophosphoric acid) is that the depolymerization reaction already starts at lower temperatures and can be performed under atmospheric conditions. A suitable concentration of catalyst used for the depolymerization of polyamide 6 to ε-caprolactam is known to the skilled person and can easily be determined by routine experimentation. If the concentration of the used catalyst is too low, the reaction rate is slow. On the contrary, if the concentration of the used catalyst is too high, the reaction is fast, but also side reaction(s) increase. Moreover, the catalyst costs are increased, which is economically disadvantageous. Preferably, the catalyst content is from 0.01 to 100 % by weight relative to the polyamide 6 contained in the depolymerization reactor. Even more preferably, the catalyst content is from 0.1 to 50 % by weight. The optimum catalyst concentration depends on the type of catalyst that is applied for the depolymerization of polyamide 6. For the catalyst orthophosphoric acid, the preferred content is from 0.1 to 25 % by weight and more preferred from 1 to 20 % by weight. The preferred content for the catalyst p-toluenesulfonic acid is from 10 to 35 % by weight and the more preferred content from 15 to 30 % by weight.

The depolymerization of polyamide 6 can be performed in a batch mode, in a semi-continuous mode or in a continuous mode, all of which are known to the skilled person. The terms "batch mode", "semi-continuous mode" and "continuous mode", as used herein, refer to the mode in which the polyamide 6 containing feedstock, i.e., the polyamide 6 rich stream, and optionally catalyst are charged to the depolymerization reactor and the mode in which the residual material is discharged from the depolymerization reactor.

In a preferred embodiment, the polyamide 6 depolymerization is performed in the batch mode. In the batch mode, the feedstock, i.e., the polyamide 6 rich stream, and optionally catalyst are initially charged to the depolymerization reactor. Subsequently, superheated steam is charged to the depolymerization reactor and ε-caprolactam is discharged from the depolymerization reactor as vapor stream comprising ε-caprolactam and water. Next, charging of the superheated steam to the depolymerization reactor is interrupted. After optionally removing residual material from the depolymerization reactor, a new cycle is started by charging feedstock (and optionally catalyst) to the depolymerization reactor. In a preferred embodiment, residual material is not removed in between every cycle.

In a particular advantageous embodiment, the polyamide 6 depolymerization is performed in the continuous mode. In the continuous mode, the polyamide 6 containing feedstock (and optionally catalyst) is continuously charged to the depolymerization reactor. At the same time, superheated steam is continuously charged to the depolymerization reactor and ε-caprolactam is continuously discharged from the depolymerization reactor as vapor stream comprising ε-caprolactam and water. Optionally, the catalyst is continuously or intermittently charged to the depolymerization reactor. In addition, residual material is continuously discharged from the depolymerization reactor. Preferably, the polyamide 6 rich stream is charged as a melt. Preferably, the catalyst is charged as a melt, a slurry or a solution.

In another preferred embodiment, the polyamide 6 depolymerization is performed in the semi-continuous mode. In the semi-continuous mode, polyamide 6 containing feedstock (and optionally catalyst) is intermittently charged to the depolymerization reactor, while superheated steam is continuously charged to the depolymerization reactor and ε-caprolactam is continuously discharged from the depolymerization reactor as a vapor stream comprising ε-caprolactam and water. Residual material is intermittently discharged from the depolymerization reactor in the semi-continuous mode of polyamide 6 depolymerization.

### The recovering step c.3)

In the recovery section [D], ε-caprolactam is recovered from the ε-caprolactam comprising stream that is discharged from the depolymerization section [C]. This stream comprises ε-caprolactam and impurities. Preferably, this recovery is performed by a (partial) condensation of the ε-caprolactam comprising stream.

Preferably, in case no solvent is charged to the depolymerization section [C], the ε-caprolactam that is obtained by condensation is dissolved in water, whereby an ε-caprolactam-rich phase is obtained. This ε-caprolactam-rich phase also comprises impurities.

Preferably, in case water is charged to the depolymerization section [C] as solvent, the ε-caprolactam comprising stream that is discharged from the depolymerization section [C] comprises ε-caprolactam, water and impurities. The ε-caprolactam can be separated from the remaining components of the vapor stream by sending the vapor stream from the depolymerization reactor, preferably overhead, to a (preferably partial) condenser to obtain a condensate containing ε-caprolactam. Preferably, the ε-caprolactam is separated from the remaining components of the vapor stream by sending the product stream from the depolymerization reactor, preferably overhead, to a distillation column from which a water-rich phase is obtained as top product and a ε-caprolactam-rich phase as bottom product.

The ε-caprolactam recovered in the recovery section [D] is crude since it contains impurities such as polyamide 6 decomposition products and/or other impurities stemming from (decomposition products of) non-polyamide 6 components of the polyamide 6 and polyether polyurethane comprising material and/or reactions with impurities. The crude ε-caprolactam that is recovered in step c.3) comprises water and ε-caprolactam, preferably it is an aqueous solution comprising ε-caprolactam. Thus, the crude ε-caprolactam recovered in the recovery section [D] requires additional purification to yield high purity ε-caprolactam. "Crude" as used herein can therefore be defined as being less pure, i.e., containing more impurities, than the purified ε-caprolactam obtained after purification in a purification section [E].

Preferably, the crude ε-caprolactam comprises ε-caprolactam in the range of from 6% to 95 %, more preferably from 20 % to 90 %, and most preferably from 35 % to 80 % by weight. The remainder is mainly water.

### The purifying step c.4)

Preferably, the process of the invention is performed further in a plant that further comprises

-    a purification section [E],

and wherein the process further comprises the steps of:
c.4) purifying the crude ε-caprolactam obtained in the recovery section [D] in the purification section [E] to obtain purified ε-caprolactam wherein the purification comprises the steps of:

(i) Extracting the crude ε-caprolactam with an organic solvent, whereby an aqueous phase and an organic phase are obtained, and wherein the organic phase comprises the organic solvent, ε-caprolactam and impurities.

(ii) Optionally, switching the solvent by replacing the organic solvent at least partially with water, whereby an aqueous phase comprising water, ε-caprolactam and impurities with lower- or higher-boiling points than ε-caprolactam is obtained. The solvent switch step c.4)(ii) can in particular be selected from a process based on back-extraction with water, and a process based on solvent swap distillation, in which the organic solvent is distilled off and water is charged.

(iii) Obtaining purified ε-caprolactam by distillative removal of impurities with lower- or higher-boiling points than ε-caprolactam.

In step c.4), the crude ε-caprolactam which is obtained in the recovery section [D] is purified in the purification section [E] to yield high purity ε-caprolactam.

Optionally, the crude ε-caprolactam is filtered before being charged to the purification section [E]. The filtration ensures the removal of undissolved impurities which could otherwise hinder the further purification process.

Purified ε-caprolactam is obtained from the crude ε-caprolactam by first extracting in step (i) the crude ε-caprolactam with an organic solvent, whereby an aqueous phase and an organic phase comprising the organic solvent, ε-caprolactam and impurities are obtained. The organic solvent with which the crude ε-caprolactam is extracted is preferably an aromatic hydrocarbon, a halogenated hydrocarbon and/or a C4-C10 aliphatic or C4-C10 cycloaliphatic alcohol. Optionally, the organic solvent with which the crude ε-caprolactam is extracted is preferably a mixed extractant, i.e., it can consist of one or more organic solvents and optionally further diluents. Preferably, the one or more organic solvents is independently selected from an aromatic hydrocarbon, a halogenated hydrocarbon and/or a C4-C10 aliphatic or cycloaliphatic alcohol, and a C5-C8 alkane or C5-C8 cycloalkane. Particularly good purification results are achieved if the organic solvent for extraction of the crude ε-caprolactam is selected from the group consisting of cyclohexane, benzene, toluene, methylene chloride, chloroform, trichloroethane, 4-methyl-2-pentanol (a.k.a. MIBC, methyl isobutyl carbinol), 1-octanol, 2-ethylhexanol and mixtures thereof. More preferably, the organic solvent for extraction of the crude ε-caprolactam is selected from the group consisting of benzene, toluene, cyclohexane, alcohols, and mixtures thereof. Still more preferably, the organic solvent for extraction of the crude ε-caprolactam is selected from the group consisting of toluene, cyclohexane, 1-octanol, 2-ethylhexanol and mixtures thereof. Preferably, the weight ratio of organic solvent to ε-caprolactam is from 0.01:1 to 50:1, preferably from 0.05:1 to 20:1, more preferably from 0.1:1 to 10:1, and most preferably from 0.1:1 to 5:1.

In another embodiment, the extraction with organic solvent in step c.4)(i) is carried out in a counter-current operated extraction column whereby the crude ε-caprolactam to be purified is introduced at the top and the organic solvent at the bottom of the column. The extraction results in an aqueous phase and an organic phase comprising the organic solvent, ε-caprolactam and impurities.

Optionally, the organic phase comprising the organic solvent, ε-caprolactam and impurities is washed with water or with an aqueous alkaline solution before entering step c.4)(ii). If washing is performed with an aqueous alkaline solution, the alkaline solution is preferably an aqueous solution comprising an alkali metal hydroxide and/or alkali metal carbonate, preferably sodium hydroxide or potassium hydroxide. Said alkali metal hydroxide solution preferably comprises 0.5 wt.% to 2.0 wt.% of sodium hydroxide or potassium hydroxide.

The skilled person can determine by routine experimentation the amount of water or aqueous alkaline solution necessary for efficient washing of the organic phase comprising the organic solvent, ε-caprolactam and impurities. Preferably, this amount is between 0.1 vol.% and 5 vol.% relative to the amount of organic solvent in the to-be-washed organic phase.

In another preferred embodiment, the washing of the organic phase comprising the organic solvent, ε-caprolactam and impurities with water or aqueous alkaline solution is carried out in a counter-current operated washing column whereby the organic phase comprising the organic solvent, ε-caprolactam and impurities is introduced at the bottom and the water or aqueous alkaline solution at the top of the column. The washing results in a washed organic phase comprising the organic solvent, ε-caprolactam and impurities and a residue-comprising phase. The residue-comprising phase then contains water, impurities and ε-caprolactam.

In a preferred embodiment, the organic phase comprising the organic solvent, ε-caprolactam and impurities that is optionally washed is subsequently extracted with water, whereby an ε-caprolactam-water phase is obtained. Preferably, this ε-caprolactam-water phase is then stripped and/or distilled to remove residual solvent. Although the amount of the water used for the recovery of ε-caprolactam can vary, the amount of the water used is 0.5 to 20 times, preferably 0.75 to 10 times, and more preferably 1 to 5 times by weight based on the recovered ε-caprolactam.

In another preferred embodiment, the extraction with water is carried out in a counter-current operated extraction column, the ε-caprolactam containing phase to be purified being introduced at the bottom and the water at the top of the column. The extraction results in an ε-caprolactam-water phase and in a solvent phase that comprises impurities. Usually the solvent phase that comprises impurities is, optionally after purification, preferably by distillation, re-used.

The ε-caprolactam-water phase, that was optionally stripped and/or distilled to remove residual solvent, is concentrated by evaporation of water so that a concentrated aqueous ε-caprolactam phase is obtained. The ε-caprolactam content of this concentrated aqueous ε-caprolactam phase is usually between 60 wt.% and 99.9 wt.% relative to the entire phase.

In another preferred embodiment, organic solvent is evaporated from the organic phase comprising organic solvent, ε-caprolactam and impurities that is optionally washed instead of being extracted with water. Any suitable evaporation vessel may be used, for example a column. Preferably the evaporation is performed in the presence of water. More preferably, the evaporation is performed as an azeotropic distillation in which case the organic solvent is evaporated as an azeotropic mixture. The evaporation results in ε-caprolactam product. Preferably, the ε-caprolactam product is an aqueous ε-caprolactam phase. The ε-caprolactam content of this aqueous ε-caprolactam phase is usually between 40 wt.% and 99.9 wt.% relative to the entire phase.

In another preferred embodiment, prior to the distillative removal in step c.4)(ii) of the process of the invention, an oxidant, e.g., potassium permanganate, sodium permanganate, and/or hydrogen peroxide, is added to the ε-caprolactam-water phase. Most preferably, potassium permanganate is used as oxidant.

Preferably, the oxidant is added as solid, as a slurry or in the form of an aqueous solution to the ε-caprolactam-water phase, so that a dilute aqueous solution is obtained during purification by oxidation. The skilled person can determine by routine experimentation the amount of oxidant necessary for efficient oxidation of the ε-caprolactam-water phase. The exact amount of oxidant is, amongst others, very much dependent on the composition of the polyamide 6 rich streams that are charged to the depolymerization section in this process of the invention. Preferably, the amount of oxidant is between 0.01 and 5 % by weight relative to the amount of ε-caprolactam dissolved in the to-be-oxidized aqueous phase.

The temperature used for oxidation of the aqueous solution in the process of the invention can vary. Preferably, oxidation of this aqueous solution prior to the distillative removal in step c.4)(ii) with an oxidant is performed at a temperature ranging from 20 °C to 85 °C, more preferably ranging from 30 °C to 80 °C, wherein the oxidant is selected from the group consisting of potassium permanganate, sodium permanganate and hydrogen peroxide and combinations thereof, in particular potassium permanganate.

The length of time used for oxidation with an oxidant can vary. Preferably, in the process of the invention, prior to the distillative removal in step c.4)(ii), oxidation of the ε-caprolactam-water phase with an oxidant is performed for 1 minute to 24 hours, more preferably for 2 minutes to 6 hours, and most preferably for 5 minutes to 2 hours.

The concentration of ε-caprolactam in the ε-caprolactam-water phase used for oxidation with an oxidant can vary. Preferably, the aqueous solution used for oxidation comprises a weight to weight ratio of ε-caprolactam to water from 5 : 1 to 1 : 5, more preferably from 3 : 1 to 1 : 3 and, most preferably from 2 : 1 to 1 : 2. Optionally, prior to the addition of the oxidant to the aqueous phase, the weight to weight ratio of ε-caprolactam to water is adapted. Preferably, the weight to weight ratio of ε-caprolactam to water is adapted by either addition of water or by removal of water.

In case potassium permanganate and/or sodium permanganate is used as oxidant, solid manganese(IV) oxide ($MnO_2$) particles are formed as reaction product. The skilled person can determine by routine experimentation the optimal solid-liquid filtration procedure for efficient removal of solid manganese(IV) oxide particles from the aqueous phase after oxidation. The usage of filter aids, like activated carbon or kieselguhr particles, to improve the filtration procedure are in this regard common practice.

The aqueous ε-caprolactam phase is optionally hydrogenated and if the aqueous ε-caprolactam phase is hydrogenated, then preferably in the presence of a hydrogenation catalyst known per se. The hydrogenation can be carried out as for example described in EP635487.

Optionally water is evaporated from the, optionally hydrogenated, aqueous ε-caprolactam phase. Preferably, water is evaporated from the, optionally hydrogenated, aqueous ε-caprolactam phase. Following the hydrogenation and/or evaporation of water, the aqueous ε-caprolactam phase is distilled to recover high purity ε-caprolactam and a distillation residue.

In step c.4)(iii) of the process of the invention, the optionally washed organic phase comprising the organic solvent, ε-caprolactam and impurities is subsequently distilled. Preferably, distillation of the optionally washed organic phase comprising the organic solvent, ε-caprolactam and impurities is carried out at a reduced pressure. In one embodiment, the distillation is effected at a pressure of less than 350 kPa, preferably less than 50 kPa, more preferably less than 20 kPa and most preferably less than 10 kPa. Preferably, the distillation temperature at the bottom of the distillation column is between 100 °C and 200 °C and more preferably between 110 °C and 180 °C. The distillation includes the separation of low-boiling organic impurities (having a lower boiling point than ε-caprolactam) and/or separating high-boiling organic impurities (having a higher boiling point than ε-caprolactam) from ε-caprolactam.

In a preferred embodiment, prior to the distillative removal in step c.4)(iii), alkali metal hydroxide, preferably NaOH, is added to the ε-caprolactam comprising phase. Preferably, the amount of NaOH that is added ranges from 0.5 to 100 mmol per kg ε-caprolactam, and more preferably from 2 to 80 mmol per kg ε-caprolactam. This leads to a particularly effective distillative removal of impurities with lower and higher boiling points than ε-caprolactam.

In another preferred embodiment, crude ε-caprolactam obtained from a Beckmann rearrangement of cyclohexanone oxime is also purified in the purification section apart from the crude ε-caprolactam that is recovered in the recovery section. This has the advantage that by introducing recycled ε-caprolactam according to the present invention, the carbon footprint of a plant producing ε-caprolactam *de novo* from a Beckmann rearrangement of cyclohexanone oxime can be reduced.

The high purity ε-caprolactam obtained according to the method of the invention can be used to make polyamide 6 using processes well-known to the skilled person. The polyamide 6 may then be used in all known materials, including engineering materials, fibers and films.

The recovery of polyether polyurethane (steps d.1) and d.2)):

The polyether polyurethane rich stream which is obtained during the separation step b) is used for the recovery of polyether polyurethane in the separation section [B], wherein the polyether polyurethane rich stream is a solution comprising a solvent and polyether polyurethane (step d.1)).

In step d.2) of the process of the invention, the recovered polyether polyurethane is discharged from the separation section [B]. Optionally, the recovered polyether polyurethane is dried prior to being discharged from the separation section [B].

Steps d.1) and d.2) can be replaced by steps b.3) and b.4). A preferred embodiment of the process of the invention is therefore performed in a plant that comprises

- a separation section [B] comprising a dissolution section [$\alpha$], a washing section [$\beta$], a precipitation section [$\gamma$], and a solvent distillation section [$\delta$],
- a depolymerization section [C], and
- a recovery section [D],

and comprises the following steps:

a) charging polyamide 6 and polyether polyurethane comprising material to the separation section [B];

b.1) charging an organic solvent, and polyamide 6 and polyether polyurethane comprising material to the dissolution section [$\alpha$] of the separation section [B];

b.2) dissolving polyether polyurethane from the polyamide 6 and polyether polyurethane comprising material in an organic solvent in the dissolution section [$\alpha$], so that a polyether polyurethane rich stream comprising organic solvent and dissolved polyether polyurethane, and a stream comprising non-dissolved polyamide 6 are obtained and discharging the obtained streams from the dissolution section [$\alpha$];

b.3) charging a second solvent and said polyether polyurethane rich stream comprising organic solvent and dissolved polyether polyurethane to the precipitation section [$\gamma$] of the separation section [B] so that polyether polyurethane is precipitated from a mixture comprising organic solvent and second solvent;

b.4) recovering said precipitated polyether polyurethane from said mixture comprising organic solvent and second solvent and discharging said precipitated polyether polyurethane from the precipitation section [$\gamma$];

b.5) discharging the mixture comprising organic solvent and second solvent from which precipitated polyether polyurethane has been recovered in step b.4) from the precipitation section [$\gamma$] and charging said mixture to the solvent distillation section [$\delta$] of the separation section [B];

b.6) charging a third solvent and the stream comprising non-dissolved polyamide 6 to the washing section [$\beta$] of the separation section [B];

b.7) washing the stream comprising non-dissolved polyamide 6 with the third solvent in the washing section [$\beta$], so that a polyamide 6 rich stream and a mixture comprising organic solvent and third solvent are obtained;

b.8) discharging said polyamide 6 rich stream from the washing section [$\beta$];

b.9) discharging the mixture comprising organic solvent and third solvent that is obtained in step b.7) from the washing section [$\beta$] and charging said mixture to the solvent distillation section [$\delta$] of the separation section [B];

b.10) separating organic solvent from the second solvent and the third solvent by distillation in the solvent distillation section [$\delta$] and discharging the second solvent, the third solvent, and the separated organic solvent from the solvent distillation section [$\delta$].

c.1) discharging the polyamide 6 rich stream from separation section [B] and charging the polyamide 6 rich stream to depolymerization section [C] wherein the polyamide 6 content of the polyamide 6 rich stream is at least 85 %, preferably at least 90 % by weight on dry-weight basis;

c.2) depolymerizing the polyamide 6 in the polyamide 6 rich stream in the depolymerization section [C] at a temperature ranging from 180 °C to 400 °C so that an $\epsilon$-caprolactam comprising stream is obtained and discharging the obtained $\epsilon$-caprolactam comprising stream from the depolymerization section [C];

c.3) recovering crude $\epsilon$-caprolactam from said $\epsilon$-caprolactam comprising stream in the recovery section [D].

The plant

The invention also provides a plant in which the above-described process of the invention can be carried out. The plant is a chemical plant. All plant features specifically described in connection with the plant below therefore also correspond to specific embodiments of the process of the invention and vice versa. Thus, the plant is suitable for carrying out the process of the invention and it is to be understood that what has been described in connection with the process of the invention equally applies to the plant embodiments.

The plant can be a laboratory setup as in the examples. Preferably, however, the plant is an industrial scale plant. "Industrial scale" means that the plant has a production capacity for ε-caprolactam (i.e., is in principle capable of producing the same in an amount of) of at least 500 tons/year if operated all the time.

The plant of the invention is suitable for the production of purified ε-caprolactam and polyether polyurethane from polyamide 6 and polyether polyurethane comprising material and comprises at least the following three sections: a separation section [B], a depolymerization section [C], and a recovery section [D], and further optionally, a pre-treatment section [A] and optionally, a purification section [E]. These sections, and thereby the plant, is configured to carry out the process of the invention described above.

In addition, the plant of the invention can comprise a pre-treatment section [A], which can comprise a mechanical size reduction section to fragment the polyamide 6 and polyether polyurethane comprising material into pieces and/or a cleaning section to wash the polyamide 6 and polyether polyurethane comprising material. Cleaning includes both washing and separation of foreign materials from the polyamide 6 and polyether polyurethane comprising material. The separation of foreign materials can be done both manually (handpicking) and mechanically (e.g., density separation, and magnetic separation). Both manual and mechanical devices, like brushes, can help with the washing in the cleaning section. The washing is preferably carried out by an additional effect of friction. Different types of industrial washing systems are available on the market, like rotary plastic washers and (high speed) friction washers. The mechanical size reduction section comprises equipment for the mechanical fragmentation of the polyamide 6 and polyether polyurethane comprising material into pieces. Non-limiting examples of this fragmentation equipment are a cutter, a puncher, a shredder, a mill, a grinder, or a chipper.

The separation section [B] can comprise the following four sections: a dissolution section [α], a washing section [β], a precipitation section [γ], and a solvent distillation section [δ]. These sections, and thereby the plant, is configured to carry out the process of the invention described above.

The dissolution section [α] comprises one or more dissolution units that are operated in series and/or in parallel, and one or more separation units that are operated in series and/or in parallel. The optionally cleaned and/or fragmented pieces of polyamide 6 and polyether polyurethane comprising material is fed to the dissolution unit(s) as a solid or as a melt, preferably as a solid. The optionally cleaned and/or fragmented pieces of polyamide 6 and polyether polyurethane comprising material is optionally dried prior to being fed to the dissolution section [α]. The dissolution unit(s) is/are equipped with inlets for charging separated organic solvent and fresh organic solvent. The dissolution unit(s) is/are equipped with one or more outlets for discharging the mixture obtained in the dissolution unit(s). This mixture comprises non-dissolved polyamide 6 and organic solvent in which polyether polyurethane is dissolved. Preferably, the dissolution unit(s) is/are closed. This has the advantage that the emission of organic solvent to the environment is reduced. Preferably, the dissolution unit(s) is/are equipped with means to control the temperature in the unit(s) (e.g., steam coils, steam tracing, electrical tracing). Optionally, the temperature of the separated organic solvent and fresh organic solvent is adjusted (e.g., in a heat exchanger) before being charged to the dissolution section [α].

Good contact between organic solvent and the polyamide 6 and polyether polyurethane comprising material is essential for an effective operation. Such contact may be achieved by various means known generally in the art. Improved contact can be achieved by mechanical friction. Mechanical friction in turn can be achieved, e.g., by agitation with a combination of rotating paddles and static fins.

Dissolution units suitable for the dissolution of a polymer in an organic solvent are known to the man skilled in the art. A stirred vessel is an example of such a dissolution unit.

In the separation unit(s) the mixture that is discharged from the dissolution unit(s) is separated into a polyether polyurethane rich stream comprising organic solvent and dissolved polyether polyurethane, and a stream comprising non-dissolved polyamide 6. The polyether polyurethane rich stream comprising organic solvent and dissolved polyether polyurethane is discharged via a discharge line and charged to precipitation section [γ]. The stream comprising non-dissolved polyamide 6 is discharged via a discharge line and charged to washing section [β]. It is advantageous that the amount of organic solvent in the stream comprising non-dissolved polyamide 6 is low in order to facilitate the washing in the washing section [β].

Separation unit(s) suitable for the separation of (non-dissolved) polymer and an organic solvent are known to the man skilled in the art. A centrifuge and a filter are examples of such a separation unit.

Preferably, a dissolution unit and a separation unit are combined in one piece of equipment. This leads to a process intensification.

The washing section [β] comprises equipment for washing the stream comprising non-dissolved polyamide 6 with a third solvent to obtain a polyamide 6 rich stream and a mixture comprising organic solvent and third solvent. The purpose of the washing section [β] is to remove the organic solvent to a large extent. Optionally, the washing section [β] comprises equipment for drying the polyamide 6 rich stream. The mixture comprising organic solvent and third solvent is discharged from the washing section [β] and is charged to the solvent distillation section [δ]. Herein, "charged to the solvent distillation section [δ]" also includes indirect means, i.e., additional steps/sections in between. Preferably, the mixture comprising organic solvent and third solvent that is discharged from the washing section [β] is completely or partly charged to the precipitation section [γ] before being charged to the solvent distillation section [δ].

Optionally after being dried, the polyamide 6 rich stream is discharged from the washing section [β] and is charged to the depolymerization section [C].

Good contact between the third solvent and the polyamide 6 rich stream is essential for an effective operation. Such contact can be achieved by various means known to the skilled person. One means is a (continuous) centrifuge that is fed with the polyamide 6 rich stream and to which the third solvent is charged as washing solvent.

The distillation section [δ] comprises one or more distillation columns that are operated in series and/or in parallel. Distillation columns can be operated in a batch-wise, a semi-continuous or in a continuous mode. The choice between these modes of operation is very much dependent on the scale of operation. In general, batch-wise operation of distillation columns is more suited for treating low volume feed streams. Continuous operation of distillation columns is more suited for treating large volume feed streams.

The exact lay-out of the distillation section [δ] also depends on the nature of the organic solvent, the second solvent and the third solvent.

The distillation section [δ] is charged with the mixture comprising organic solvent and second solvent from which precipitated polyether polyurethane has been recovered and the mixture comprising organic solvent and third solvent that was discharged from the washing section [β]. Separated organic solvent is discharged from the distillation section [δ] and is charged to the dissolution section [α]. The second solvent and the third solvent are discharged from the distillation section [δ] and are optionally charged to precipitation section [γ] and to the washing section [β], respectively. Residues are discharged from the distillation section [δ]. Optionally, the residues are incinerated, whereby energy is recovered. Finally, degradation products of the used solvents are discharged from the distillation section [δ]. Acetic acid and dimethylamine are examples of degradation products of organic solvent N,N-dimethylacetamide (DMAc).

The depolymerization section [C] comprises one or more depolymerization reactors that are operated in series and/or in parallel. The polyamide 6 rich stream is fed to the reactor as a solid or as a melt, preferably as a melt. This feeding may be achieved by using an extruder, gear pump, or other means known in the art.

During production, a depolymerization reactor is at least partially filled with polyamide 6-containing feedstock, residual material, ε-caprolactam (and optionally catalyst). The depolymerization reactor can have any desirable form. Preferred reactor types are stirred and non-stirred bubble column reactors, stirred reactors and extruder type reactors.

The depolymerization reactor must be equipped with facilities for feeding of the polyamide 6 rich stream, the superheated steam and optionally the catalyst. In addition, the depolymerization reactor must be equipped with facilities for discharging the vapor stream comprising ε-caprolactam and water, and the residual material.

Good contact between steam and the reactor content is essential for an effective operation. Such contact may be achieved by various means known by the skilled person. As an example, steam may be sparged through the material using a multiplicity of inlets provided by, e.g., a steam distributor. Improved contact can also be achieved by including mechanical agitation in the reactor. The mechanical agitation, can be achieved, e.g., by using a combination of rotating paddles and static fins.

Preferably, depolymerization is complete in 0.5 to 6 hours.

If superheated steam at high temperatures is not available on a production site, it has to be made on purpose by superheating of available steam from a boiler in a so-called superheater.

The recovery section [D] comprises one or more (preferably partial) condensers to which the vapor stream comprising ε-caprolactam and water is charged. Such a (partial) condenser can have any desirable form. Preferably, a condenser is a distillation column from which a water-rich phase is obtained as top-product and crude ε-caprolactam as bottom product.

The purification section [E] comprises one or more pieces of extraction equipment, one or more pieces of distillation equipment, optionally, one or more pieces of solvent switch equipment, optionally, an oxidation section, and optionally, a hydrogenation section, to which the crude ε-caprolactam is charged and from which high purity ε-caprolactam is discharged.

The crude ε-caprolactam and the organic solvent are charged to the extraction equipment and an organic phase comprising the organic solvent, ε-caprolactam and impurities, and an aqueous phase comprising water and impurities are discharged. The extraction equipment is selected from mixer-settler extractors, extraction columns and centrifugal extractors and combinations thereof. Preferably, extraction equipment is a static or an agitated extraction column, like KARR® Columns, SCHEIBEL® Columns, rotating disc contactors (RDC), pulsed columns, sieve trays (static) columns, random packing (static) columns, and structured packing (SMVP) (static) columns.

Water and an organic phase comprising the organic solvent, ε-caprolactam and impurities are charged to the solvent switch equipment and an organic solvent comprising phase and an ε-caprolactam-water phase comprising the water, ε-caprolactam, and impurities are discharged. The solvent switch equipment for processes based on back-extraction is selected from mixer-settler extractors, extraction columns and centrifugal extractors and combinations thereof. Preferably, equipment for back-extraction is a static or an agitated extraction column, like KARR® Columns, SCHEIBEL® Columns, rotating disc contactors (RDC), pulsed columns, sieve trays (static) columns, random packing (static) columns, and structured packing (static) columns.

The solvent switch equipment for processes based on solvent swap distillation is selected from sieve trays distillation columns, random packing distillation columns, and structured packing distillation columns. Preferably, the distillation column is equipped with a reboiler, a condenser and equipment for reflux. The distillation column can be operated at atmospheric pressure, sub-atmospheric pressure or super-atmospheric pressure. Preferably, water is charged to the upper part of the distillation column and the aqueous phase comprising water, ε-caprolactam and impurities with lower- or higher-boiling points than ε-caprolactam is discharged from the lower part of the distillation column.

The optionally present oxidation section comprises one or more oxidation reactors that are operated in series and/or in parallel. An oxidant and the ε-caprolactam-water phase comprising water, ε-caprolactam, and impurities are charged to the oxidation section. The oxidant can be charged as solid, as a slurry or in the form of an aqueous solution. Preferably, the oxidant is charged as an aqueous solution. In case potassium or sodium permanganate is applied as oxidant then the oxidation section also comprises a filtration section. The oxidation reactor can have any desirable form. Preferred reactor types are stirred and non-stirred reactors and packed column type reactors. The oxidation reactor must be equipped with facilities for feeding of the aqueous phase comprising water, ε-caprolactam and impurities with lower- or higher-boiling points than ε-caprolactam, and the oxidant. In addition, the oxidation reactor must be equipped with facilities for discharging the oxidized ε-caprolactam-water phase comprising the water, ε-caprolactam, and impurities, and optionally formed solid manganese(IV) oxide ($MnO_2$) particles. Usually, the oxidation is performed at a temperature ranging from 20 °C to 85 °C and at atmospheric conditions.

The optionally present solid manganese(IV) oxide ($MnO_2$) particles can be removed by settling or by solid-liquid filtration, preferably by solid-liquid filtration. The usage of filter aids, like activated carbon particles and kieselguhr, to improve the filtration procedure are common practice. Filter systems suitable for the separation of solid manganese(IV) oxide particles are known to the skilled person. To such a filter system a suspension of the oxidized ε-caprolactam-water phase comprising water, ε-caprolactam and impurities, and the solid manganese(IV) oxide particles are charged and the filtered oxidized ε-caprolactam-water phase comprising the water, ε-caprolactam and impurities is discharged. Generally, the solid manganese(IV) oxide particles are retained in the filter system. Typically, such a filter system is operated in a semi-continuous mode so that the suspension and the filtered phase are continuously charged and continuously discharged, respectively, while the separated solids are collected in the filter system. From time to time, the charging of the suspension is interrupted and the collected solids are removed from the filter system.

The purification of the crude ε-caprolactam to obtain purified ε-caprolactam can comprise a hydrogenation with a heterogeneous catalyst, in which case the plant will comprise a hydrogenation section. Preferably, the catalyst comprises nickel or palladium.

The hydrogenation section comprises one or more hydrogenation reactors that are operated in series and/or in parallel. The hydrogenation can be performed in a three-phase system (gas, liquid, solid) that consists of an aqueous ε-caprolactam mixture, gaseous hydrogen and heterogeneous hydrogenation catalyst. Alternatively, the hydrogenation can be performed in a two-phase system (liquid, solid) that consists of an aqueous ε-caprolactam mixture, that is completely or partially saturated with hydrogen, and heterogeneous hydrogenation catalyst. Dissolution of the hydrogen in the water-ε-caprolactam mixture can be effected by any process that is known to one skilled in the art. Preferably, the mixture is contacted with hydrogen in an absorber or in a mixer in which a constant hydrogen pressure is maintained. Intensive contact between the hydrogen and the mixture will ensure that the hydrogen dissolves in the mixture. Such a process is preferably carried out continuously. The hydrogen-containing mixture is subsequently contacted with the hydrogenation catalyst for example in a separate reactor.

The heterogeneous catalyst can be contacted with the hydrogen-containing reaction mixture in various ways. Hydrogenation may for instance take place in a stirred tank reactor in which the catalyst particles are suspended in the mixture to be hydrogenated (slurry phase process). In such a slurry phase process the catalyst particles and the purified mixture must be separated in an additional process step after the hydrogenation reaction, for instance by means of filtration. Preferably, the catalyst comprises palladium or nickel.

Alternatively, the hydrogenation can be effected in a fixed-bed reactor with the catalyst being fixed in the reactor, so that the additional step for separation of the catalyst and reaction mixture can be dispensed with. Preferably, the fixed bed is consisting of a supported palladium or nickel catalyst.

The hydrogenation temperature is generally between 20 and 160°C. The hydrogenation pressure is generally between 0.1 and 15 MPa.

To the distillation equipment the ε-caprolactam-water phase comprising water, ε-caprolactam and impurities is charged and high purity ε-caprolactam, water and impurities (i.e., low-boiling organic impurities having a lower boiling point than ε-caprolactam and organic high-boiling impurities having a higher boiling point than ε-caprolactam) are discharged. The distillation equipment is selected from sieve trays distillation columns, random packing distillation columns, structured packing distillation columns, horizontal and vertical (falling and climbing) film evaporators. Preferably, the distillation columns are equipped with a reboiler, a condenser and equipment for reflux. The distillation equipment can be operated at atmospheric pressure, sub-atmospheric pressure or super-atmospheric pressure, preferably at sub-atmospheric pressure.

Preferably, the distillation includes the separation of water, low-boiling organic impurities (having a lower boiling point than ε-caprolactam) from ε-caprolactam and/or separating organic high-boiling impurities (having a higher boiling point than ε-caprolactam) from ε-caprolactam. The distilling thus includes in a first step the separation of water from ε-caprolactam as top product while ε-caprolactam containing low-boiling impurities and high-boiling impurities are removed as a bottom product. In a second step, low-boiling impurities are removed from ε-caprolactam as top product while ε-caprolactam containing high-boiling impurities is obtained as bottom product. In a third step, high purity ε-caprolactam is separated out as a top product while leaving as a bottom product a distillation residue comprising ε-caprolactam and high boiling impurities. Optionally, the first step and the second step are combined.

Typically, prior to the distillative removal of water and impurities, alkali metal hydroxide, preferably NaOH, is added to the oxidized ε-caprolactam-water phase comprising the water, ε-caprolactam and impurities. Preferably, the amount of NaOH that is added ranges from 0.5 to 100 mmol per kg ε-caprolactam, more preferably from 2 to 25 mmol per kg ε-caprolactam. This leads to a particularly effective distillative removal of impurities with lower and higher boiling points than ε-caprolactam in the subsequent distillation.

The process of the invention can be operated in a continuous, semi-continuous or batch-wise fashion. Accordingly, also the plant of the invention can be configured to allow for one or more of these operating modes. In a preferred embodiment, the plant is configured to operate the process of the invention in a continuous or semi-continuous fashion. However, a discontinuous process is also possible. For example, the plant of the invention does not need to contain all sections described herein in one location. In particular, the pre-treatment section [A] can be located at a first location, while the separation section [B], the depolymerization section [C], the recovery section [D] and the purification section [E] are located at a second location. Similarly, also a mechanical size reduction section as part of the pre-treatment section [A], can be located at a first location, while a cleaning section as part of pre-treatment section [A] can be located at a second location. The separation section [B], the depolymerization section [C], the recovery section [D] and the purification section [E] can then even be located at a third location.

Optionally, the cleaning section is split in two or more segments that are optionally all located at different locations. E.g., a first segment of a cleaning section as part of pre-treatment section [A] can be located at a first location, a mechanical size reduction section as part of the pre-treatment section [A] can be located at a second location, while a second segment of cleaning section as part of pre-treatment section [A] can be located at a third location. The separation section [B], the depolymerization section [C], the recovery section [D] and the purification section [E] are located at a fourth location.

Optionally, the separation section [B] can be located at a location that differs from the location(s) of pre-treatment section [A], the depolymerization section [C], the recovery section [D] and the purification section [E]. Preferably, the separation section [B] can be located near a location whereby the recovered polyether polyurethane is re-used.

The product

The process of the invention allows producing ε-caprolactam and polyether polyurethane, which meet the specification for high demanding applications, and at the same time the process is particularly environmentally friendly due to its lowered product carbon footprint and the use of waste as starting material.

In a preferred embodiment, the ε-caprolactam obtained by the process of the invention fulfils one or more of the following specifications, wherein the parameters and measurement methods are defined as in the Example section herein below:

| PAN: | max. 5 |
| E290: | max. 0.05 |
| VB: | max. 0.5 mmol/kg |
| Alkalinity: | max. 0.1 mmol/kg |

In preferred embodiments, the polyether polyurethane obtained by the process of the invention can be re-used as such or in combination with virgin polyether polyurethane in the production of textiles.

The ε-caprolactam produced by the process of the invention is also particularly economically and environmentally friendly. This is evident from the much lower carbon footprint of the ε-caprolactam produced by the process of the invention as compared to classically produced ε-caprolactam (e.g., by Beckmann rearrangement of cyclohexanone oxime).

The polyether polyurethane produced by the process of the invention is also particularly economically and environmentally friendly. Again, this is evident from the much lower carbon footprint of the polyether polyurethane produced by the process of the invention as compared to classically produced polyether polyurethane, e.g., by reaction of polyether polyols and diisocyanate monomer.

The environmental impact of a product is generally expressed as the product carbon footprint or short as 'carbon footprint'. The carbon footprint of a product is defined as the total emissions caused by the formation of that product, expressed as ton carbon dioxide equivalent per ton product. The carbon footprint of a product is amongst others depending on the feedstock, auxiliary materials, energy consumption, energy sources, production process and process efficiencies. Quantification of the carbon footprint of a product can be done as described in, e.g., the European Standard EN ISO 14040:2006 ("Environmental management - Life cycle assessment - Principles and framework).

Product carbon footprint calculations can be done both in-house or by external (preferably) certified organizations. These organizations verify and certify the product carbon footprint calculations based on e.g., LCA standard ISO 14040.

J. Hong and X. Xu ("Environmental impact assessment of caprolactam production - a case study in China"; J. of Cleaner production 27 (2012) 103-108; DOI: 10.1016/j.jclepro.2011.12.037) reported that the potential impact of "virgin" $\varepsilon$-caprolactam obtained via Beckmann rearrangement of cyclohexanone oxime on global warming is 7.5 ton $CO_2$ equivalent per ton $\varepsilon$-caprolactam (which is equal to 7.5 kg $CO_2$ equivalent per kg $\varepsilon$-caprolactam), in case coal-based electricity and steam generation are involved. If natural gas-based electricity and steam generation are involved, the potential impact of virgin $\varepsilon$-caprolactam on global warming of the $\varepsilon$-caprolactam production process will be reduced to 6.4 ton $CO_2$ equivalent per ton $\varepsilon$-caprolactam (which is equal to 6.4 kg $CO_2$ equivalent per kg $\varepsilon$-caprolactam).

The product carbon footprint of $\varepsilon$-caprolactam that is obtained according to the process of the invention is much lower than the one of *de novo* synthesized, or "virgin" $\varepsilon$-caprolactam. The product carbon footprint of the $\varepsilon$-caprolactam that is obtained according to the process of the invention is less than 5.0 kg, preferably less than 4.0 kg and most preferably less than 3.0 kg $CO_2$ equivalent per kg $\varepsilon$-caprolactam.

N.M. van der Velden, M.K. Patel and J.G. Vogtländer (Table 7 in: "LCA benchmarking study on textiles made of cotton, polyester, nylon, acryl, or elastane", Int J Life Cycle Assess (2014) 19:331-356; DOI: 10.1007/s11367-013-0626-9) reported that the potential impact of virgin elastane production on global warming is equal to 4.836 kg $CO_2$ equivalent per kg elastane.

The product carbon footprint of polyether polyurethane that is obtained according to the process of the invention is much lower than the one of *de novo* synthesized, or "virgin" polyether polyurethane. The product carbon footprint of the polyether polyurethane that is obtained according to the process of the invention is less than 3.0 kg, preferably less than 2.0 kg $CO_2$, and most preferably less than 1.0 kg $CO_2$ equivalent per kg polyether polyurethane.

## BRIEF DESCRIPTION OF THE DRAWINGS

In the following, the invention will be more fully described with reference to the Figures, which depict certain embodiments of the invention. The invention, however, is as defined in the claims and as generally described herein. It should not be limited to the embodiments shown for illustrative purposes in the Figures below.

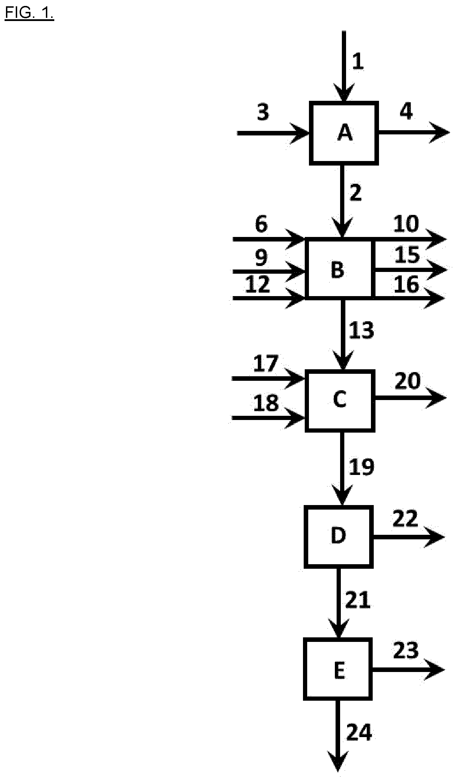

FIG. 1 is a schematic of the process of the invention for the recovery of both purified $\varepsilon$-caprolactam and polyether polyurethane from polyamide 6 and polyether polyurethane comprising material, comprising processing steps performed in optionally a pre-treatment section [A], a separation section [B], a depolymerization section [C], a recovery section [D] and optionally a purification section [E].

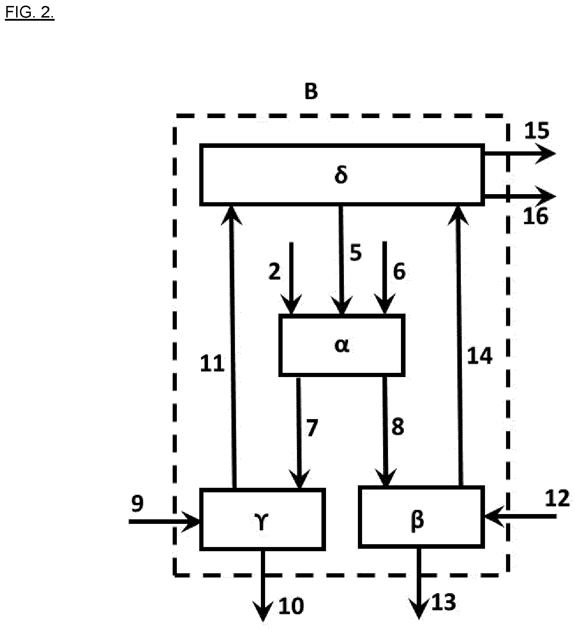

FIG. 2 illustrates an embodiment of the separation section [B], in which the polyamide 6 and polyether polyurethane comprising material is separated to obtain a polyamide 6 rich stream and a polyether polyurethane rich stream. The embodiment consists of a dissolution section [$\alpha$], a washing section [$\beta$], a precipitation section [$\gamma$], and a solvent distillation section [$\delta$].

## DETAILED DESCRIPTION OF THE DRAWINGS

The process of the invention is schematically illustrated in FIG. 1. The process is carried out in the following plant sections:

In the optional pre-treatment section [A] polyamide 6 and polyether polyurethane comprising material [1] is cleaned and/or fragmented by mechanical size reduction to obtain cleaned and/or fragmented pieces of polyamide 6 and polyether polyurethane comprising material [2] that are discharged. Optionally, in the pre-treatment section [A], in which the polyamide 6 and polyether polyurethane comprising material [1] is cleaned by removal of foreign materials and/or by washing with a washing solvent [3], foreign materials and contaminated washing solvent [4] are obtained that are discharged. Removal of foreign materials can be done prior and/or after the washing with a washing solvent [3]. Cleaning can be done prior and/or after the fragmentation of the polyamide 6 and polyether polyurethane comprising material [1]. Optionally, the polyamide 6 and polyether polyurethane comprising material [2] is dried after the cleaning step and prior to being charged to the separation section [B];

In the separation section [B] the optionally cleaned and/or fragmented pieces of polyamide 6 and polyether polyurethane comprising material [2] are separated in a polyamide 6 rich stream [13] and a polyether polyurethane rich stream. Preferably, the separation of polyamide 6 and polyether polyurethane comprising material [2] in a polyamide 6 rich stream [13] and a polyether polyurethane rich stream is achieved by selective dissolution of polyether polyurethane in an organic solvent. Optionally, fresh organic solvent is charged to separation section [B] via line [6]. Optionally, a second solvent is charged to separation section [B] via line [9], which is used to precipitate polyether polyurethane from the polyether polyurethane rich stream so that precipitated polyether polyurethane [10] is obtained. Optionally, precipitated polyether polyurethane [10] is dried prior to being discharged from the separation section [B]. Optionally, a third solvent is charged to separation section [B] via line [12], which is used to wash the polyamide 6 rich stream [13] prior to being discharged from the separation section [B]. Optionally, the polyamide 6 rich stream [13] is dried prior to being discharged from the separation section [B]. Optionally, separation section [B] comprises a section (preferably a solvent distillation section) that separates off the second solvent and the third solvent [15] and residues [16], which are discharged from separation section [B];

In depolymerization section [C] the polyamide 6 rich stream [13] is depolymerized so that an ε-caprolactam comprising stream [19] is obtained which is discharged from the depolymerization section [C]. In addition, residual material [20] is discharged. Superheated steam [17] and optionally a catalyst [18] are charged to the depolymerization section [C]. Optionally, the polyamide 6 rich stream [13] is melted before being charged to depolymerization section [C];

In recovery section [D] crude ε-caprolactam [21] is recovered from the ε-caprolactam comprising stream [19] that is discharged from the depolymerization section [C]. In addition, an aqueous phase [22] is discharged from the recovery section [C];

In the optional purification section [E] crude ε-caprolactam [21] that is discharged from recovery section [D] is purified to yield high purity ε-caprolactam [24]. Water and impurities [23] are also discharged from the purification section [E].

FIG. 2 depicts an embodiment of the separation section [B] (area enclosed by the dashed line), in which the polyamide 6 and polyether polyurethane comprising material is separated to obtain a polyamide 6 rich stream and a polyether polyurethane rich stream. This embodiment comprises the following sections:

In the dissolution section [α] polyether polyurethane is dissolved in an organic solvent from the optionally cleaned and/or fragmented pieces of polyamide 6 and polyether polyurethane comprising material [2] to obtain a polyether polyurethane rich stream comprising organic solvent and dissolved polyether polyurethane [7] and a stream comprising non-dissolved polyamide 6 [8]. These are discharged from dissolution section [α]. The organic solvent is the separated organic solvent [5] to which optionally fresh organic solvent [6] is added;

In the precipitation section [γ] a second solvent [9] and the polyether polyurethane rich stream comprising organic solvent and dissolved polyether polyurethane [7] are mixed so that polyether polyurethane is precipitated from a mixture comprising organic solvent and second solvent. The precipitated polyether polyurethane is recovered (e.g., by filtration or centrifugation) from the mixture comprising organic solvent and second solvent. The precipitated polyether polyurethane [10] that is recovered is discharged from precipitation section [γ]. Optionally, the precipitated polyether polyurethane [10] is dried after the recovery step and prior to being discharged from the precipitation section [γ]. The mixture comprising organic solvent and second solvent from which precipitated polyether polyurethane has been recovered [11] is discharged from the precipitation section [γ] and charged to the solvent distillation section [δ];

In the washing section [β] the stream comprising non-dissolved polyamide 6 [8] is washed with a third solvent [12] to obtain a polyamide 6 rich stream [13] and a mixture comprising organic solvent and third solvent [14]. The polyamide 6 rich stream [13] is discharged from the washing section [β]. Optionally, the polyamide 6 rich stream [13] is dried after the washing step and prior to being discharged from the washing section [β]. The mixture comprising organic solvent and third solvent [14] is discharged from the washing section [β] and optionally charged to the solvent distillation section [δ];

In the solvent distillation section [δ] the mixture comprising organic solvent and second solvent from which precipitated polyether polyurethane has been recovered [11] and optionally the mixture comprising organic solvent and third solvent [14] are distilled to obtain separated organic solvent [5], second solvent and third solvent [15], and residues [16]. Optionally, the second solvent and the third solvent are both water, in which case separated organic solvent [5] is dried organic solvent. The separated organic solvent [5] is charged to dissolution section [α].

## EXAMPLES

The following examples serve to explain the invention in more detail, in particular with regard to certain forms of the invention. The examples, however, are not intended to limit the present disclosure.

ε-caprolactam, that can be used for all major polymerization applications, without dilution with purer qualities of ε-caprolactam, fulfils all of the following specifications:

| PAN: | max. 5 |
| E290: | max. 0.05 |
| VB: | max. 0.5 mmol/kg |
| Alkalinity: | max. 0.1 mmol/kg |

The parameters and measurement methods are defined as follows:

PAN : ISO DIS 8660-Plastics-Determination of permanganate index of caprolactam- Spectrometric method, revision of first edition ISO 8660; 1988,
E290 : ISO 7059-caprolactam for industrial use-determination of absorbance at a wavelength of 290 nm.

Volatile bases (VB) ISO 8661-Caprolactam for industrial use-Determination of volatile bases content-Titrimetric method after distillation.

Alkalinity of $\varepsilon$-caprolactam product: the alkalinity is determined by titration at a temperature of 25 °C using a Tashiro indicator in a 1 : 2 ratio of 0.1 wt./$v_{Ethanol}$% Methylene blue : 0.1 wt./$v_{Ethanol}$% Methyl red, which is grey at its end point. A flask containing water and indicator is first titrated to grey, then X grams of an aqueous $\varepsilon$-caprolactam solution containing Y wt.% $\varepsilon$-caprolactam (as determined by refractive index) is added and the solution is titrated back to grey using a 0.01 N $H_2SO_4$ solution. Alkalinity is then given by:

$$\text{Alkalinity (mmol/kg } \varepsilon\text{-caprolactam)} = v * t * 1000 / (X * Y)$$

Where:

v = volume of $H_2SO_4$ solution added (ml)
t = normality of $H_2SO_4$ solution (= 0.01 N)
X = weight of sample (g)
Y = concentration $\varepsilon$-caprolactam (wt.%)

EXAMPLE 1

Pre-treatment and separation of polyamide 6 and polyether polyurethane

The polyether polyurethane content of the used polyether polyurethane containing polyamide 6 wasted fabric was 20.2 +/- 0.4 wt.% (as determined by Differential Scanning Calorimetry (DSC) Method ISO11357-3_-130 °C to 300 °C_10 °C/min_dt 1.00 s).

The polyether polyurethane containing polyamide 6 wasted fabric was cut in small pieces ranging from 5 to 20 cm$^2$ each. 150.4 grams of this cut material was dissolved in 800 grams of DMAc at a temperature of 70 °C for 1 hour under stirring. The resulting solution was vacuum filtered through a heated double walled Buchner funnel at a temperature of 70 °C, whereby filtrate 1 was obtained. The remaining non-dissolved material was further treated three times with each time 400 grams DMAc at a temperature of 70 °C for 1 hour under stirring, whereby the filtrates 2 to 4 were obtained.

The non-dissolved material on the Buchner funnel was washed with 325 grams of water at a temperature of 65 °C, whereby filtrate 5 was obtained and then the non-dissolved material was dried and afterwards weighed. The five filtrates were combined, whereby a precipitate and a clear solution were obtained. The precipitate was filtered off and washed with 200 grams of water and subsequently dried and afterwards weighed. The weight fraction of the dried precipitate was 20 wt.% of the total weight of the dried precipitate and the dried non-dissolved material, which was almost equal to the weight of the starting material.

Analysis by Differential Scanning Calorimetry (DSC) revealed that the polyether polyurethane content of the dry precipitate was circa 100 wt.% (polyamide 6 was not detected). Based on DSC analysis, the nylon 6 content of the dry non-dissolved material was circa 100 wt.% (polyether polyurethane was not detected).

The recovered polyether polyurethane precipitate can be re-used as-such or in combination with virgin polyether polyurethane in the production of textiles.

This EXAMPLE shows that polyether polyurethane and polyamide 6 can be separated from polyether polyurethane containing polyamide 6 wasted fabric by selective extraction, precipitation of the dissolved polyether polyurethane and washing of the non-dissolved polyamide 6. After drying, the recovered precipitate consists of almost pure polyether polyurethane and the polyether polyurethane content of the non-dissolved material is much lower than that of the polyether polyurethane containing polyamide 6 wasted fabric, that was used as starting material.

EXAMPLE 2

Pre-treatment and separation of polyamide 6 and polyether polyurethane

The polyether polyurethane content of the used polyether polyurethane containing polyamide 6 wasted fabric was 8.15 +/- 0.05 wt.% (as determined by Differential Scanning Calorimetry (DSC) Method ISO11357-3_-130 °C to 300 °C_10 °C/min_dt 1.00 s).

The procedure of EXAMPLE 1 was followed except that now 254.1 grams of the polyether polyurethane containing polyamide 6 wasted fabric was dissolved in 1200 grams of DMAc and after filtration 475 grams of DMAc and 350 grams of water were used for washing of the remaining non-dissolved material on the Buchner funnel. The precipitate was filtered off and washed with 200 grams of water and subsequently dried. The non-dissolved material on the Buchner funnel was washed with 1000 grams of water then also dried and afterwards weighed. The weight fraction of the dry precipitate was 8 wt.% of the total weight of the dry precipitate and the dry non-dissolved material, which was almost equal to the weight of the starting material.

Analysis by Differential Scanning Calorimetry (DSC) revealed that the polyether polyurethane content of the dry precipitate was circa 100 wt.% (polyamide 6 was not detected). The polyamide 6 content and the polyether polyurethane content of the dry non-dissolved material were circa 99 wt.% and less than 1 wt.%, respectively (DSC analysis).

The recovered polyether polyurethane precipitate can be re-used as-such or in combination with virgin polyether polyurethane in the production of textiles.

This EXAMPLE shows that polyether polyurethane and polyamide 6 can be separated from polyether polyurethane containing polyamide 6 wasted fabric by selective extraction, precipitation of the dissolved polyether polyurethane and washing of the non-dissolved polyamide 6. After drying, the recovered precipitate consists of almost pure polyether polyurethane and the polyether polyurethane content of the non-dissolved material is much lower than that of the starting material polyether polyurethane containing polyamide 6 wasted fabric.

EXAMPLE 3

Depolymerization of polyamide 6 and recovery of ε-caprolactam

The dry non-dissolved material obtained in EXAMPLE 1 was first densified before it was charged to the depolymerization reactor. The dry non-dissolved material was melted under nitrogen at 237 °C and forced through a perforated metal plate. The obtained strands were cooled down to room temperature and cut into pellets. The diameter and the length of the resulting pellets were 3 mm and 1 cm, respectively.

48 grams of these polyamide 6 containing pellets and 14 grams of 20 wt.% phosphoric acid were charged to a Premex high pressure autoclave. First, the reactor content was heated under nitrogen and subsequently superheated steam was injected continuously at a rate of 4 grams per minute during the 120-minute reaction. The temperature and the pressure in the reactor were maintained at 260 °C and 0.11 MPa, respectively. During the reaction a vapor stream was continuously discharged from the reactor and was cooled down to ca. 20 °C, whereby an ε-caprolactam and water comprising condensate was obtained.

The condensate that composed of 41 grams of ε-caprolactam, most of the remainder being water, was concentrated by evaporation in a rotavap (rotary evaporator) that was operated under vacuum (9.5 kPa; water bath temperature was ca. 65 °C) to an ε-caprolactam concentration of 51.5 wt.%. (This mixture, crude ε-caprolactam, is the mixture to be purified.)

The specifications of the crude ε-caprolactam were:

| | |
|---|---|
| PAN: | 389 |
| E290: | 3.42 |

This EXAMPLE shows that crude ε-caprolactam can be obtained by depolymerization of polyamide 6 that has been obtained by extractive separation from polyether polyurethane containing polyamide 6 wasted fabric.

EXAMPLE 4

Depolymerization of polyamide 6 and recovery of ε-caprolactam

The procedure of EXAMPLE 3 was followed except that now 48 grams of pellets made from dry non-dissolved material obtained in EXAMPLE 2 and 14 grams of 20 wt.% phosphoric acid were charged to a Premex high pressure autoclave.

The condensate that composed of 33 grams of ε-caprolactam, most of the remainder being water, was concentrated by evaporation in a rotavap that was operated under vacuum (9.5 kPa; water bath temperature was ca. 65 °C) to an ε-caprolactam concentration of 64.6 wt.%. (This mixture, crude ε-caprolactam, is the mixture to be purified.)

The specifications of the crude ε-caprolactam were:

| | |
|---|---|
| PAN: | 214 |
| E290: | 2.68 |

This EXAMPLE shows that crude ε-caprolactam can be obtained by depolymerization of polyamide 6 that has been obtained by extractive separation from polyether polyurethane containing polyamide 6 wasted fabric.

COMPARATIVE EXPERIMENT 1

Depolymerization of polyether polyurethane

The procedure of EXAMPLE 3 was followed except that now 37.6 grams of polyether polyurethane fibers (same material as included in the wasted fabrics used in EXAMPLE 1 and EXAMPLE 2) and 14 grams of 20 wt.% phosphoric acid were charged to a Premex high pressure autoclave.

10 minutes after starting the injection of the superheated steam the experiment had to be stopped, because the line for discharging the ε-caprolactam and water comprising vapor stream was clogged by an insoluble material.

From this COMPARATIVE EXPERIMENT, it can be concluded that depolymerization of polyether polyurethane containing polyamide 6 wasted fabric without removal of polyether polyurethane prior to the depolymerization can give operational issues.

COMPARATIVE EXPERIMENT 2

Depolymerization of polyether polyurethane containing polyamide 6 wasted fabric and recovery of ε-caprolactam

The procedure of EXAMPLE 3 was followed except that now 48 grams of pellets made from polyether polyurethane containing polyamide 6 wasted fabric with a polyether polyurethane content of 20.2 +/- 0.4 wt.% (same feedstock as used in EXAMPLE 1) and 14 grams of 20 wt.% phosphoric acid were charged to a Premex high pressure autoclave.

The condensate that composed of 26 grams of ε-caprolactam, most of the remainder being water, was concentrated by evaporation in a rotavap that was operated under vacuum (9.5 kPa; water bath temperature was ca. 65 °C) to an ε-caprolactam concentration of 47.0 wt.%. (This mixture, crude ε-caprolactam, is the mixture to be purified.)

The specifications of the crude ε-caprolactam were:

| | |
|---|---|
| PAN: | 352 |
| E290: | 3.60 |

Observations:

- The obtained condensate before concentration did contain an unknown precipitate
- Fouling of the internal walls of the Premex high pressure autoclave was seen after this depolymerization experiment

From this COMPARATIVE EXPERIMENT, it can be concluded that depolymerization of polyether polyurethane containing polyamide 6 wasted fabric without removal of polyether polyurethane prior to the depolymerization can give operational issues. Another observation is that the ε-caprolactam yield in this COMPARATIVE EXPERIMENT is much lower than in EXAMPLE 3 (with pre-treated polyether polyurethane containing polyamide 6 wasted fabric as feed), whereby the ε-caprolactam yield is defined as the ratio of the weight of ε-caprolactam in the condensate to the weight of polyamide 6 in the feed that is charged to the Premex high pressure autoclave.

COMPARATIVE EXPERIMENT 3

Purification by distillation

75 mmol of aqueous sodium hydroxide per kg ε-caprolactam was then added to a part of the crude ε-caprolactam obtained in COMPARATIVE EXPERIMENT 2. This mixture was then distilled by reducing the pressure in steps. ε-caprolactam was distilled at 300 Pa.
The specifications of the distilled ε-caprolactam were:

| | |
|---|---|
| PAN: | 31 |
| E290: | 3.08 |
| VB: | 2.45 mmol/kg |
| Alkalinity: | 3.13 mmol/kg |

This COMPARATIVE EXPERIMENT shows that the quality of ε-caprolactam that is obtained by depolymerization of polyether polyurethane containing polyamide 6 wasted fabric (without pre-treatment in a separation section, in which the polyamide 6 and polyether polyurethane comprising material is separated in a polyamide 6 rich stream and a polyether polyurethane rich stream) and subsequently concentrated and purified by distillation is very poor as it does not meet any of the required specifications for major polymerization applications.

COMPARATIVE EXPERIMENT 4

Purification by distillation

75 mmol of aqueous sodium hydroxide per kg ε-caprolactam was then added to a part of the crude ε-caprolactam obtained in EXAMPLE 3. This mixture was then distilled by reducing the pressure in steps. ε-caprolactam was distilled at 300 Pa.
The specifications of the distilled ε-caprolactam were:

| | |
|---|---|
| PAN: | 33 |
| E290: | 0.39 |
| VB: | 2.12 mmol/kg |
| Alkalinity: | 2.24 mmol/kg |

From this COMPARATIVE EXPERIMENT, it can be concluded that purification of crude ε-caprolactam by just distillation is insufficient to meet the required specifications for major polymerization applications.

COMPARATIVE EXPERIMENT 5

Purification by permanganate treatment and distillation

The crude ε-caprolactam obtained in EXAMPLE 3 was treated with 0.2 wt.% $KMnO_4$ with regard to ε-caprolactam at 50 °C for 2 hours. The solids formed were then removed from the oxidized reaction product by means of a filtration.
The ε-caprolactam in the oxidized reaction product was after addition of 75 mmol of aqueous sodium hydroxide per kg ε-caprolactam further purified by distillation as described in COMPARATIVE EXAMPLE 4.
The specifications of the distilled ε-caprolactam were:

| | |
|---|---|
| PAN: | 23 |
| E290: | 0.63 |
| VB: | 2.26 |
| Alkalinity: | 1.69 |

From this COMPARATIVE EXPERIMENT, it can be concluded that purification of crude ε-caprolactam by permanganate treatment succeeded by distillation is insufficient to meet all the required specifications for major polymerization applications as quantified above.

EXAMPLE 5

Purification by extraction, oxidation and distillation

Part of the crude ε-caprolactam obtained in EXAMPLE 4 was further concentrated by evaporation in a rotavap that was operated under vacuum (9.5 kPa; water bath temperature was ca. 65 °C) to an ε-caprolactam concentration of 37.9 wt.%. (This mixture, concentrated crude ε-caprolactam, is the mixture to be purified.)

The concentrated crude ε-caprolactam was 11 times batch-wise extracted with a solvent mixture of 4-methyl-2-pentanol (50 wt.%) / cyclohexane (50 wt.%) at a temperature of ca. 25 °C. Total amount of extraction solvent used was 3.22 grams of 4 methyl-2-pentanol / cyclohexane per gram of concentrated crude ε-caprolactam.

The concentrated extracts were 7 times batch-wise extracted with water at a temperature of ca. 25 °C. Total amount of water used was 5.67 grams of water / gram of recovered ε-caprolactam.

The combined aqueous extracts were treated with 0.1 wt.% $KMnO_4$ with regard to ε-caprolactam at 50 °C for 2 hours. The solids formed were then removed from the oxidized reaction product by means of a filtration.

The resulting mixture was concentrated by evaporation in a rotavap that was operated under vacuum (9.5 kPa, water bath temperature was ca. 65 °C) to an ε-caprolactam concentration of 49.7 wt.%. The ε-caprolactam in the resulting concentrated aqueous solution was after addition of 75 mmol of aqueous sodium hydroxide per kg ε-caprolactam further purified by distillation as described in COMPARATIVE EXAMPLE 4.

The specifications of the distilled ε-caprolactam were:

| | |
|---|---|
| PAN: | 0.9 |
| E290: | 0.03 |
| VB: | 0.07 |
| Alkalinity: | 0.10 |

From this EXPERIMENT, it can be concluded that purified ε-caprolactam that meets the required specifications for major polymerization applications can be obtained from depolymerized polyether polyurethane containing polyamide 6 wasted fabric from which polyether polyurethane has been removed by extraction and has been purified by extraction, oxidation and distillation.

EXPERIMENT 6

Calculation of carbon footprint of purified ε-caprolactam and polyether polyurethane

A continuous process according to the invention for the production of purified ε-caprolactam and polyether polyurethane from polyether polyurethane containing polyamide 6 wasted fabric was simulated. The process included:

- Cutting polyether polyurethane containing polyamide 6 wasted fabric in small pieces
- Selective extraction of polyether polyurethane with DMAc;
- Separation of non-dissolved polyamide 6 and polyether polyurethane rich stream by centrifugation;
- Washing of non-dissolved polyamide 6 with water;
- Separation of washed non-dissolved polyamide 6 and aqueous extract by centrifugation;
- Precipitation of polyether polyurethane from the polyether polyurethane rich stream by addition of the aqueous extract obtained above;
- Separation of precipitated polyether polyurethane by filtration from DMAc-water mixture;
- Recovery of DMAc and water from DMAc-water mixture obtained above;
- Drying of filtered polyether polyurethane;
- Drying of washed non-dissolved polyamide 6;
- Melting and pelletization of washed non-dissolved polyamide 6;
- Depolymerization of polyamide 6 under influence of $H_3PO_4$ and superheated steam;
- Recovery of crude ε-caprolactam (80 wt.% ε-caprolactam) by partial condensation of vapors discharged from depolymerization reactor;
- Counter-current extraction of concentrated crude ε-caprolactam with toluene;

- Washing of organic extract with diluted caustic solution;
- Counter-current extraction of washed organic extract with water;
- Treating with $KMnO_4$ and filtration of the formed solids;
- Evaporative concentration of aqueous extract;
- Addition of caustic;
- Recovery of pure $\varepsilon$-caprolactam by vacuum distillation.

The carbon footprints of purified $\varepsilon$-caprolactam and polyether polyurethane were calculated based on the consumption figures of raw materials, and utilities of the above described process are based on data originating from ecoinvent version 3.7.1. The distribution of the environmental impact between the products purified $\varepsilon$-caprolactam and polyether polyurethane in the pre-treatment section and in the separation section was based on the weight ratio of these products.

The outcome revealed that the product carbon footprint of purified $\varepsilon$-caprolactam obtained from polyether polyurethane containing polyamide 6 wasted fabric is less than 3.0 ton $CO_2$ eq./ton of $\varepsilon$-caprolactam and less than 1.0 ton $CO_2$ eq./ton of polyether polyurethane.

While the invention has been described and with reference to specific embodiments thereof, it will be apparent to those of ordinary skill in the art that various changes and modifications, including (semi-)continuous operations and upscaling to commercial scale, can be made therein without departing from the spirit and scope thereof.

**Claims**

1.  A process for recovering $\varepsilon$-caprolactam and polyether polyurethane from a polyamide 6 and polyether polyurethane comprising material in a plant, wherein the plant comprises

    - a separation section [B],
    - a depolymerization section [C], and
    - a recovery section [D],

    and wherein the process comprises the steps of:

    a) charging polyamide 6 and polyether polyurethane comprising material to the separation section [B];
    b) separating the polyamide 6 and polyether polyurethane comprising material in a polyamide 6 rich stream and a polyether polyurethane rich stream in the separation section [B] by selective dissolution of the polyether polyurethane in an organic solvent at a temperature below 100 °C, preferably at a temperature ranging from 0 °C to 100 °C, more preferably at a temperature ranging from 10 °C to 90 °C, even more preferably at a temperature ranging from 10 °C to 80 °C, and most preferably at a temperature ranging from 20 °C to 75 °C, wherein the polyether polyurethane rich stream is a solution comprising the organic solvent and polyether polyurethane;
    c.1) discharging the polyamide 6 rich stream from separation section [B] and charging the polyamide 6 rich stream to depolymerization section [C] wherein the polyamide 6 content of the polyamide 6 rich stream is at least 85 % by weight on dry-weight basis;
    c.2) depolymerizing the polyamide 6 in the polyamide 6 rich stream in the depolymerization section [C] at a temperature ranging from 180 °C to 400 °C so that an $\varepsilon$-caprolactam comprising stream is obtained and discharging the obtained $\varepsilon$-caprolactam comprising stream from the depolymerization section [C];
    c.3) recovering crude $\varepsilon$-caprolactam from said $\varepsilon$-caprolactam comprising stream in the recovery section [D];
    d.1) recovering polyether polyurethane from the polyether polyurethane rich stream in the separation section [B]; and
    d.2) discharging said recovered polyether polyurethane from the separation section [B] wherein the polyether polyurethane content of the discharged stream is at least 85 % by weight on dry-weight basis.

2.  A process according to claim 1, wherein the plant comprises

    - a purification section [E],

    and wherein the process further comprises the steps of:
    c.4) purifying the crude $\varepsilon$-caprolactam obtained in the recovery section [D] in the purification section [E] to obtain purified $\varepsilon$-caprolactam wherein the purification comprises the steps of

(i) extracting the crude ε-caprolactam with an organic solvent so that an aqueous phase and an organic phase are obtained, and wherein the organic phase comprises the organic solvent, ε-caprolactam and impurities;

(ii) optionally, switching the solvent by replacing the organic solvent at least partially with water, whereby an aqueous phase comprising water, ε-caprolactam and impurities with lower- or higher-boiling points than ε-caprolactam is obtained; and

(iii) obtaining purified ε-caprolactam by distillative removal of impurities with lower- or higher-boiling points than ε-caprolactam.

3. A process according to claim 2, wherein the separation section [B] comprises:

- a dissolution section [α],
- a washing section [β],
- a precipitation section [γ], and
- a solvent distillation section [δ],

and wherein the process comprises the following steps in the separation section [B]:

b.1) charging an organic solvent, and polyamide 6 and polyether polyurethane comprising material to the dissolution section [α];

b.2) dissolving polyether polyurethane from the polyamide 6 and polyether polyurethane comprising material in an organic solvent in the dissolution section [α], so that a polyether polyurethane rich stream comprising organic solvent and dissolved polyether polyurethane, and a stream comprising non-dissolved polyamide 6 are obtained and discharging the obtained streams from the dissolution section [α];

b.3) charging a second solvent and said polyether polyurethane rich stream comprising organic solvent and dissolved polyether polyurethane to the precipitation section [γ] so that polyether polyurethane is precipitated from a mixture comprising organic solvent and second solvent;

b.4) recovering said precipitated polyether polyurethane from said mixture comprising organic solvent and second solvent and discharging said precipitated polyether polyurethane from the precipitation section [γ];

b.5) discharging the mixture comprising organic solvent and second solvent from which precipitated polyether polyurethane has been recovered in step b.4) from the precipitation section [γ] and charging said mixture to the solvent distillation section [δ];

b.6) charging a third solvent and the stream comprising non-dissolved polyamide 6 to the washing section [β];

b.7) washing the stream comprising non-dissolved polyamide 6 with the third solvent in the washing section [β], so that a polyamide 6 rich stream and a mixture comprising organic solvent and third solvent are obtained;

b.8) discharging said polyamide 6 rich stream from the washing section [β];

b.9) discharging the mixture comprising organic solvent and third solvent that is obtained in step b.7) from the washing section [β] and charging said mixture partly or completely to the solvent distillation section [δ];

b.10) separating organic solvent from the second solvent and the third solvent by distillation in the solvent distillation section [δ] and discharging the second solvent, the third solvent, and the separated organic solvent from the solvent distillation section [δ].

4. A process according to claim 3, wherein the separated organic solvent that is discharged from the solvent distillation section [δ] in step b.10) is charged to the dissolution section [α] in step b.1).

5. A process according to any one of claims 1 to 4, wherein

(i) the polyamide 6 rich stream that is discharged from the separation section [B] is dried prior to being charged to the depolymerization section [C]; and/or

(ii) the depolymerization in step c.2) is performed in the presence of water so that the ε-caprolactam comprising stream is a vapor stream comprising ε-caprolactam and water in a weight to weight ratio of from 1:1 to 1:50, in particular from 1:2 to 1:15, from 1:2 to 1:10 or from 1:3 to 1:8; and/or

(iii) the depolymerization section [C] is charged with superheated steam having a temperature ranging from 220 °C to 575 °C, in particular from 275 °C to 500 °C.

6. A process according to any one of claims 3 to 5, wherein the recovered polyether polyurethane that is discharged from the precipitation section [γ] in step b.4) is re-used in the production of textiles.

7. A process according to any one of claims 1 to 6, wherein the plant further comprises

- a pre-treatment section [A],

and wherein prior to step a) the polyamide 6 and polyether polyurethane comprising material is subjected to a pre-treatment in the pre-treatment section [A], in particular to a cleaning in a cleaning section and/or to a mechanical size reduction in a mechanical size reduction section.

8.  A process according to any one of claims 1 to 7, wherein the organic solvent is dimethylacetamide.

9.  A process according to any one of claims 1 to 8, wherein the second solvent and the third solvent are the same solvent, more preferably water.

10. A process according to any one of claims 1 to 8, wherein the second solvent in step b.3) is partly or completely the mixture comprising organic solvent and third solvent that is obtained in step b.7) from the washing section [β].

11. A process according to any one of claims 1 to 10, wherein the second solvent and the third solvent that are discharged from the solvent distillation section [δ], optionally after separation from each other, are re-used in the precipitation section [γ] and/or in the washing section [β].

12. A process according to any one of claims 4 to 11, wherein degradation products of the organic solvent are removed prior to charging the separated organic solvent that is discharged from the solvent distillation section [δ] in step b.10) to the dissolution section [α] in step b.1).

13. A plant for the production of purified ε-caprolactam and polyether polyurethane from polyamide 6 and polyether polyurethane comprising material, wherein the plant comprises

- optionally, a pre-treatment section [A],
- a separation section [B],
- a depolymerization section [C],
- a recovery section [D], and
- optionally, a purification section [E], and

wherein the chemical plant is configured for carrying out a process as defined in any one of claims 1 to 12.

14. Polyether polyurethane obtained by a process as defined in any one of claims 1 to 12, wherein the polyether polyurethane has a product carbon footprint of less than 1.0 kg $CO_2$ per kg recovered polyether polyurethane.

15. ε-caprolactam obtained by a process as defined in any one of claims 1 to 12, wherein the ε-caprolactam has a product carbon footprint of less than 3.0 kg $CO_2$ per kg purified ε-caprolactam.

FIG. 1.

FIG. 2.

Europäisches
Patentamt

European
Patent Office

Office européen
des brevets

# EUROPEAN SEARCH REPORT

**Application Number**

EP 22 18 4117

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GONG CAIHONG ET AL: "Simple process for separation and recycling of nylon 6 and polyurethane components from waste nylon 6/polyurethane debris", TEXTILE RESEARCH JOURNAL, vol. 91, no. 1-2, 17 June 2020 (2020-06-17), pages 18-27, XP93006676, GB ISSN: 0040-5175, DOI: 10.1177/0040517520931893 Retrieved from the Internet: URL:http://journals.sagepub.com/doi/full-xml/10.1177/0040517520931893> | 13-15 | INV. C08J11/08 C08J11/12 C08J11/14 |
| Y | * Title, Abstract, Scheme 1, page 19, right, lines 11-13 and 17-18, page 23, right, lines 37-39, Figure 4 (IR) for nylon and Figure 6 (NMR) for the urethane. * | 1-12 | |
| Y | YIN YUNJIE ET AL: "Removal of spandex from nylon/spandex blended fabrics by selective polymer degradation", TEXTILE RESEARCH JOURNAL, vol. 84, no. 1, 21 May 2013 (2013-05-21), pages 16-27, XP93006635, GB ISSN: 0040-5175, DOI: 10.1177/0040517513487790 Retrieved from the Internet: URL:http://journals.sagepub.com/doi/full-xml/10.1177/0040517513487790> * p17, left, lines 14-18 * | 8 | **TECHNICAL FIELDS SEARCHED (IPC)**<br><br>C08J<br>C07D |
| A | WO 2021/021031 A1 (LAI TRILLION [TH]) 4 February 2021 (2021-02-04) * the whole document * | 1-15 | |
| | -/-- | | |

The present search report has been drawn up for all claims

| Place of search | Date of completion of the search | Examiner |
|---|---|---|
| The Hague | 23 December 2022 | Mooibroek, Tiddo |

EPO FORM 1503 03.82 (P04C01)

Europäisches
Patentamt

European
Patent Office

Office européen
des brevets

**EUROPEAN SEARCH REPORT**

Application Number

EP 22 18 4117

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2022/115602 A1 (REGENERATED TEXTILE IND LLC [US]) 2 June 2022 (2022-06-02) * the whole document * | 1-15 | |
| A | US 2014/255255 A1 (HEILBERG RONALDO DANIEL [BR]) 11 September 2014 (2014-09-11) * the whole document * | 1-15 | |
| A | JP 2011 088943 A (TORAY INDUSTRIES) 6 May 2011 (2011-05-06) * the whole document * | 1-15 | |
| A | US 6 830 715 B1 (REINEHR ULRICH [DE] ET AL) 14 December 2004 (2004-12-14) * the whole document * | 1-15 | |
| A | CA 771 086 A (DU PONT) 7 November 1967 (1967-11-07) * the whole document * | 1-15 | |
| Y | US 5 948 908 A (SIFNIADES STYLIANOS [US] ET AL) 7 September 1999 (1999-09-07) * column 6, line 40 – line 49; examples 1, 2, 6-8 * | 1-7,9-12 | TECHNICAL FIELDS SEARCHED (IPC) |
| A | WO 2022/129022 A1 (BASF SE [DE]) 23 June 2022 (2022-06-23) * the whole document * | 1-15 | |
| A | EP 0 670 308 A1 (BASF AG [DE]) 6 September 1995 (1995-09-06) * the whole document * | 1-15 | |
| A | US 5 990 306 A (MAYER RICHARD EUGENE [US] ET AL) 23 November 1999 (1999-11-23) * column 1, line 23 – line 24 * | 1-15 | |

-/--

The present search report has been drawn up for all claims

| Place of search | Date of completion of the search | Examiner |
|---|---|---|
| The Hague | 23 December 2022 | Mooibroek, Tiddo |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

## EUROPEAN SEARCH REPORT

Application Number

EP 22 18 4117

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 1 173 419 B1 (DSM IP ASSETS BV [NL]) 2 November 2006 (2006-11-02) * the whole document * | 1-15 | |
| | ‑‑‑‑‑ | | |

TECHNICAL FIELDS
SEARCHED        (IPC)

The present search report has been drawn up for all claims

| Place of search | Date of completion of the search | Examiner |
|---|---|---|
| The Hague | 23 December 2022 | Mooibroek, Tiddo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

## ANNEX TO THE EUROPEAN SEARCH REPORT
## ON EUROPEAN PATENT APPLICATION NO.

EP 22 18 4117

This annex lists the patent family members relating to the patent documents cited in the above-mentioned European search report.
The members are as contained in the European Patent Office EDP file on
The European Patent Office is in no way liable for these particulars which are merely given for the purpose of information.

23-12-2022

| Patent document cited in search report | | Publication date | Patent family member(s) | | Publication date |
|---|---|---|---|---|---|
| WO 2021021031 A1 | | 04-02-2021 | NONE | | |
| WO 2022115602 A1 | | 02-06-2022 | NONE | | |
| US 2014255255 A1 | | 11-09-2014 | BR | PI1104721 A2 | 18-08-2015 |
| | | | EP | 2596932 A2 | 29-05-2013 |
| | | | US | 2014255255 A1 | 11-09-2014 |
| | | | WO | 2013075184 A1 | 30-05-2013 |
| JP 2011088943 A | | 06-05-2011 | NONE | | |
| US 6830715 B1 | | 14-12-2004 | AU | 3154500 A | 14-09-2000 |
| | | | BR | 0008485 A | 29-01-2002 |
| | | | CA | 2362175 A1 | 31-08-2000 |
| | | | CN | 1341170 A | 20-03-2002 |
| | | | DE | 19907830 A1 | 31-08-2000 |
| | | | EP | 1163381 A1 | 19-12-2001 |
| | | | HK | 1045179 A1 | 15-11-2002 |
| | | | JP | 2002538314 A | 12-11-2002 |
| | | | KR | 20010102353 A | 15-11-2001 |
| | | | MX | PA01008560 A | 09-04-2002 |
| | | | TW | 469305 B | 21-12-2001 |
| | | | US | 6830715 B1 | 14-12-2004 |
| | | | WO | 0050673 A1 | 31-08-2000 |
| CA 771086 A | | 07-11-1967 | NONE | | |
| US 5948908 A | | 07-09-1999 | AT | 201015 T | 15-05-2001 |
| | | | DE | 69704767 T2 | 27-02-2003 |
| | | | EP | 0892782 A1 | 27-01-1999 |
| | | | US | 5869654 A | 09-02-1999 |
| | | | US | 5948908 A | 07-09-1999 |
| | | | WO | 9734868 A1 | 25-09-1997 |
| WO 2022129022 A1 | | 23-06-2022 | NONE | | |
| EP 0670308 A1 | | 06-09-1995 | CA | 2143820 A1 | 05-09-1995 |
| | | | DE | 4407222 A1 | 07-09-1995 |
| | | | EP | 0670308 A1 | 06-09-1995 |
| | | | JP | H0841021 A | 13-02-1996 |
| | | | US | 5700358 A | 23-12-1997 |
| US 5990306 A | | 23-11-1999 | AT | 368646 T | 15-08-2007 |
| | | | CA | 2302787 A1 | 11-03-1999 |
| | | | CN | 1278792 A | 03-01-2001 |
| | | | EP | 1042284 A1 | 11-10-2000 |

EPO FORM P0459

For more details about this annex : see Official Journal of the European Patent Office, No. 12/82

**page 1 of 2**

**EP 4 306 585 A1**

**ANNEX TO THE EUROPEAN SEARCH REPORT
ON EUROPEAN PATENT APPLICATION NO.** EP 22 18 4117

This annex lists the patent family members relating to the patent documents cited in the above-mentioned European search report.
The members are as contained in the European Patent Office EDP file on
The European Patent Office is in no way liable for these particulars which are merely given for the purpose of information.

23-12-2022

| Patent document cited in search report | Publication date | Patent family member(s) | | Publication date |
|---|---|---|---|---|
| | | JP | 2001514251 A | 11-09-2001 |
| | | KR | 20010023618 A | 26-03-2001 |
| | | TW | I225482 B | 21-12-2004 |
| | | US | 5990306 A | 23-11-1999 |
| | | US | 6187917 B1 | 13-02-2001 |
| | | WO | 9911616 A1 | 11-03-1999 |
| EP 1173419 B1 | 02-11-2006 | EP | 1173419 A1 | 23-01-2002 |
| | | US | 2002038023 A1 | 28-03-2002 |
| | | WO | 0064871 A1 | 02-11-2000 |

EPO FORM P0459

For more details about this annex : see Official Journal of the European Patent Office, No. 12/82

page 2 of 2

41

**EP 4 306 585 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6830715 B1
- JP 2011088943 A
- WO 2013032408 A1
- EP 635487 A

### Non-patent literature cited in the description

- Caprolactam. Ullmann's Encyclopedia of Industrial Chemistry. 2018
- **J. HONG ; X. XU.** Environmental impact assessment of caprolactam production - a case study in China. *J. of Cleaner production,* 2012, vol. 27, 103-108
- **N.M. VAN DER VELDEN ; M.K. PATEL ; J.G. VOGTLÄNDER.** LCA benchmarking study on textiles made of cotton, polyester, nylon, acryl, or elastane. *Int J Life Cycle Assess,* 2014, vol. 19, 331-356